(19) Europäisches Patentamt
European Patent Office
Office européen des brevets

(11) EP 0 549 407 B1

(12) # FASCICULE DE BREVET EUROPEEN

(45) Date de publication et mention de la délivrance du brevet:
**25.09.1996 Bulletin 1996/39**

(51) Int Cl.[6]: **C07D 207/08**, C07D 401/04, C07D 401/06, A61K 31/40, C07D 207/48

(21) Numéro de dépôt: **92403404.4**

(22) Date de dépôt: **15.12.1992**

(54) **Nouveaux dérivés de pyrrolidine, leur procédé de préparation et les compositions pharmaceutiques qui les contiennent**

Neue Pyrrolidinderivate, Verfahren zu ihrer Herstellung und diese enthaltende Arzneimittel

Novel pyrrolidine derivatives, process for their preparation and pharmaceutical compositions containing them

(84) Etats contractants désignés:
**AT BE CH DE DK ES FR GB GR IE IT LI LU NL PT SE**

(30) Priorité: **20.12.1991 FR 9115851**

(43) Date de publication de la demande:
**30.06.1993 Bulletin 1993/26**

(73) Titulaire: **ADIR ET COMPAGNIE**
**F-92415 Courbevoie Cédex (FR)**

(72) Inventeurs:
- **Lavielle, Gilbert**
  **F-78170 La Celle Saint Cloud (FR)**
- **Hautefaye, Patrick**
  **F-77170 Servon Brie Comte Robert (FR)**
- **Laubie, Michel**
  **F-92420 Vaucbresson (FR)**
- **Verbeuren, Tony**
  **F-78540 Vernouillet (FR)**

(56) Documents cités:
**EP-A- 0 289 911** **EP-A- 0 367 130**

- **JOURNAL OF MEDICINAL CHEMISTRY, vol. 34, no. 4, 1991, WASHINGTON, US; pages 1511-1514, MAIS D.E. et al.: "Novel Synthesis and Biochemical Properties of an [125I]-Labeled Photoaffinity Probe for Thromboxane A2/Prostalglandin H2 Receptors"**
- **JOURNAL OF MEDICINAL CHEMISTRY, vol. 34, no. 9, 1991, WASHINGTON, US, pages 2882-2891, MISRA R.N. et al.: "Interphenylene 7-Oxabicyclo[2.2.1]heptane Thromboxane A2 Antagonists"**
- **JOURNAL OF MEDICINAL CHEMISTRY, vol. 34, no. 6, 1991, WASHINGTON, US, pages 1790-1797, BHAGWAT S.S. et al.: "Thromboxane Receptor Antagonism Combined with Thromboxane Synthase Inhibition."**
- **HETEROCYCLES, vol. 28, no. 2, 1989, pages 573-578, KAWADA K. et al.: "Synthesis of monocyclic analogs of a potent thromboxane receptor antagonist, (.+-.)-(5Z)-7-[3-endo-[(phenyl sulfonyl)amino]bicyclo[2.2.1]hept-2-exo-yl]hept enoic acid."**
- **PATENT ABSTRACTS OF JAPAN, vol. 13, no. 101, (C-574), 9 Mars 1989 ( SHIONOGI ) 15 Novembre 1988**

EP 0 549 407 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Printed by Jouve, 75001 PARIS (FR)

## Description

La présente invention concerne de nouveaux dérivés de la pyrrolidine, leur procédé de préparation et les compositions pharmaceutiques qui les contiennent.

Plus particulièrement, les composés décrits dans la présente invention possèdent des propriétés antithromboxane $A_2$ aussi bien en tant qu'antagonistes des récepteurs au thromboxane $A_2$ ($TXA_2$) qu'en tant qu'inhibiteurs de l'activité de l'enzyme responsable de la synthèse du thromboxane $A_2$ : la thromboxane $A_2$-synthase.

Le thromboxane $A_2$ est un métabolite de l'acide arachidonique produit par les plaquettes sanguines qui provoque une constriction importante des vaisseaux sanguins et induit l'agrégation des plaquettes. La production du thromboxane $A_2$ est augmentée dans des maladies comme l'angine de poitrine ou l'accident vasculaire cérébral et il joue un rôle très important dans tous les processus conduisant aux maladies thrombotiques.

Il était donc particulièrement intéressant de synthétiser des substances susceptibles d'inhiber les activités proagrégantes et vasoconstrictives du thromboxane $A_2$ soit en tant qu'antagonistes des récepteurs au thromboxane $A_2$, soit en tant qu'inhibiteur de la thromboxane $A_2$-synthase.

Des dérivés de pyrrolidine possédant des propriétés antithrombotiques ont été décrits dans la littérature. C'est le cas, en particulier, des composés décrits dans les brevets EP 289 911 et EP 367 130.

Les composés décrits dans la présente invention, outre le fait qu'ils soient nouveaux, possèdent des propriétés pharmacologiques nettement plus intenses que celles des autres composés décrits dans l'Art Antérieur.

Ils sont donc utiles en tant qu'antagonistes au thromboxane $A_2$ et en tant qu'inhibiteurs de la thromboxane $A_2$-synthase dans le traitement ou la prévention des maladies thrombotiques comme l'accident vasculaire cérébral, l'angine de poitrine, l'infarctus du myocarde, l'insuffisance circulatoire périphérique, les maladies liées à la formation de thrombus...

Plus spécifiquement, la présente invention concerne les composés de formule (I) :

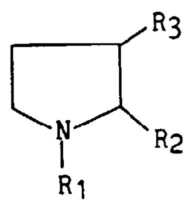

(I)

dans laquelle :

$R_1$ représente :

- un groupement alkyl ($C_1$-$C_6$) linéaire ou ramifié substitué ou non par un groupement pyridin-2-yl, pyridin-3-yl, phényl (lui-même éventuellement substitué par un ou plusieurs atomes d'halogène ou groupements alkyle ($C_1$-$C_6$) linéaire ou ramifié, alkoxy ($C_1$-$C_6$) linéaire ou ramifié, trihalogénométhyle),
- un groupement phényle substitué ou non par un ou plusieurs atomes d'halogène ou groupements alkyle ($C_1$-$C_6$) linéaire ou ramifié, alkoxy ($C_1$-$C_6$) linéaire ou ramifié, trihalogénométhyle,
- un groupement pyridyle,
- un groupement phénylsulfonyl substitué ou non sur le noyau phényle par un ou plusieurs atomes d'halogène ou groupements alkyle ($C_1$-$C_6$) linéaire ou ramifié, alkoxy ($C_1$-$C_6$) linéaire ou ramifié, trihalogénométhyle,
- un groupement acyle ($C_1$-$C_6$) linéaire ou ramifié,
- un groupement alkoxycarbonyl ($C_1$-$C_6$) linéaire ou ramifié,
- un groupement benzoyle (substitué ou non sur le noyau phényl par un ou plusieurs atomes d'halogène ou groupements hydroxy, alkyle, alkoxy, trifluorométhyle) ou pyridylcarbonyl,
- un groupement alkylaminocarbonyle ou phénylaminocarbonyle (substitué ou non sur le noyau phényl par un ou plusieurs atomes d'halogène ou groupements hydroxy, alkyle, alkoxy, trifluorométhyle),
- un groupement acylamino ou benzoylamino,

$R_2$ représente :

- un groupement phényle substitué ou non par un ou plusieurs atomes d'halogène ou groupements alkyle ($C_1$-$C_6$) linéaire ou ramifié, alkoxy ($C_1$-$C_6$) linéaire ou ramifié, hydroxy, trihalogénométhyle,

- un groupement pyridin-3-yle ou pyridin-2-yle,

$R_3$ représente l'un quelconque des groupements suivants :

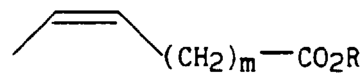

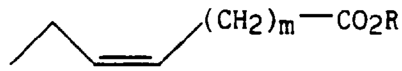

$$-(CH_2)_n-CO_2R$$

dans lesquels

m est égal à 2, 3 ou 4,

n est égal à 4, 5, 6 ou 7,

et R représente un atome d'hydrogène ou un groupement alkyle ($C_1$-$C_6$) linéaire ou ramifié,

leurs énantiomères, diastéréoisomères et épimères ainsi que leurs sels d'addition à un acide ou à une base pharmaceutiquement acceptable.

Parmi les acides pharmaceutiquement acceptables, on peut citer à titre non limitatif les acides chlorhydrique, sulfurique, tartrique, maléique, fumarique, méthane sulfonique, camphorique, etc...

Parmi les bases pharmaceutiquement acceptables, on peut citer à titre non limitatif l'hydroxyde de sodium, l'hydroxyde de potassium, la tertbutylamine, la diéthylamine, l'éthylènediamine...

L'invention s'étend également au procédé de préparation des composés de formule (I) caractérisé en ce que l'on utilise comme produit de départ une pyrrolidine de formule (II) :

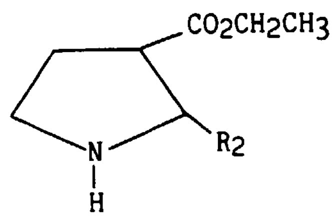

(II)

dans laquelle $R_2$ a la même signification que dans la formule (I), sous forme racémique ou dont on a préalablement séparé les isomères selon une technique classique de séparation,
que l'on fait réagir :

- soit avec de l'hydrure de lithium aluminium dans de l'éther, pour conduire à la pyrrolidine de formule (III) :

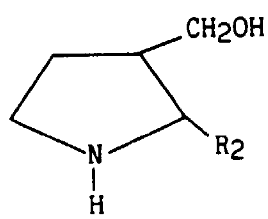

(III)

dans laquelle $R_2$ a la même signification que dans la formule (I), que l'on met alors en réaction avec un dérivé halogéné de formule $R_1X$ dans laquelle $R_1$ a la même signification que dans la formule (I) et X représente un atome d'halogène, dans du chloroforme en présence de triéthylamine ou dans de l'acétonitrile en présence de carbonate de potassium,

pour conduire à la pyrrolidine de formule (IV) :

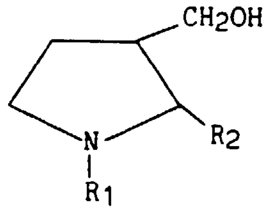

(IV)

dans laquelle $R_1$ et $R_2$ ont la même signification que dans la formule (I),

- soit avec un dérivé halogéné de formule $R_1X$ tel que défini précédemment, pour conduire à la pyrrolidine de formule (V) :

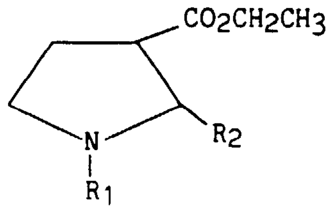

(V)

dans laquelle $R_1$ et $R_2$ ont la même signification que dans la formule (I), composé de formule (V) qui, lorsqu'il est sous forme d'un couple d'énantiomères, peut être transformé, si on le souhaite, en son autre couple d'énantiomères par réaction de l'éthanolate de sodium, en milieu éthanolique, composé de formule (V) que l'on réduit :

a ou bien :

en présence de deux équivalents d'hydrure de diisobutylaluminium dans du toluène,

pour conduire à la pyrrolidine de formule (IV) définie précédemment, composé de formule (IV), qui, quelle que soit la voie de synthèse par lequel il a été obtenu, est transformé en tosylate (Ts) correspondant de formule (VI), par réaction avec du chlorure de toluène sulfonyle en présence de pyridine,

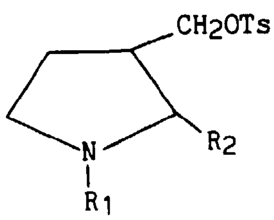

(VI)

dans laquelle $R_1$ et $R_2$ ont la même signification que dans la formule (I), sur lequel on fait réagir du cyanure de potassium en milieu diméthylsulfoxyde, pour conduire à la pyrrolidine de formule (VII) :

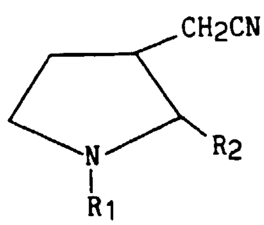

(VII)

dans laquelle $R_1$ et $R_2$ ont la même signification que dans la formule (I), que l'on réduit à l'aide d'hydrure de diisobutylaluminium dans du toluène, pour conduire à l'aldéhyde correspondant de formule (VIII) :

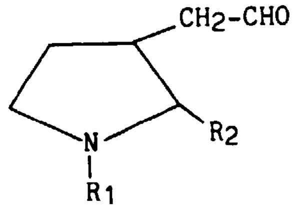

(VIII)

dans laquelle $R_1$ et $R_2$ ont la même signification que dans la formule (I),

b ou bien :

en présence d'un équivalent d'hydrure de diisobutylaluminium dans du toluène,

pour conduire à la pyrrolidine de formule (IX) :

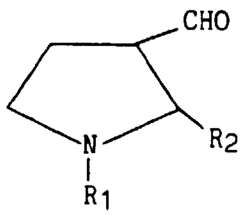

(IX)

dans laquelle $R_1$ et $R_2$ ont la même signification que dans la formule (I),
composés de formule (VIII) ou (IX) que l'on fait réagir avec un ylure de phosphore de formule (X), préparé par réaction du sel de phosphonium correspondant, en présence de terbutanolate de potassium dans du tétrahydrofurane,

$$(C_6H_5)_3\,P = CH\,\text{-}(CH_2)_m\,\text{-}CO_2H \qquad (X)$$

dans laquelle m a la même signification que dans la formule (I),
pour conduire respectivement aux composés de formule (I/a) ou (I/b), cas particulier des composés de formule (I) :

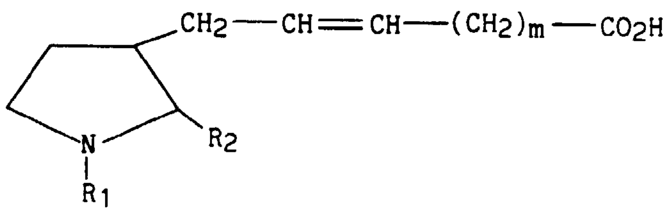

(I/a)

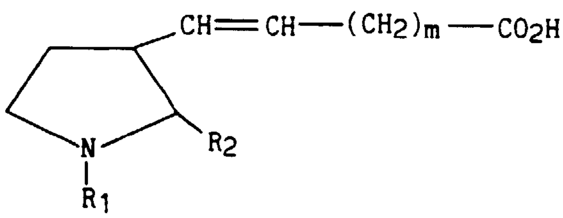

(I/b)

dans lesquelles $R_1$, $R_2$ et m ont la même signification que dans la formule (I),
composés de formule (I/a) ou (I/b)

- que l'on estérifie, si on le souhaite, pour conduire aux composés de formule ($I/a_1$) et ($I/b_1$) correspondants :

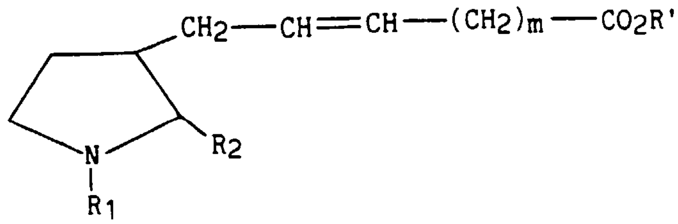

(I/a1)

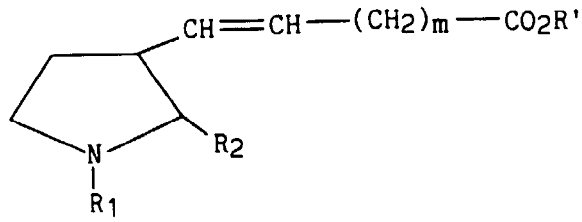

(I/b1)

dans lesquelles $R_1$ et $R_2$ ont la même signification que dans la formule (I) et R' représente un groupement alkyle ($C_1$-$C_6$) linéaire ou ramifié,

- dont on réduit éventuellement la double liaison par hydrogénation catalytique pour conduire au composé de formule (I/c), cas particulier des composés de formule (I),

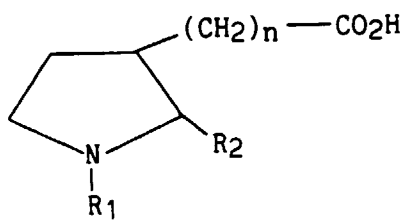

(I/c)

dans laquelle $R_1$, $R_2$ et n ont la même signification que dans la formule (I) et que l'on transforme, si on le souhaite, en ester correspondant de formule (I/$c_1$) :

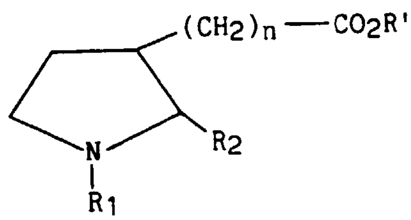

(I/c1)

dans laquelle $R_1$ et $R_2$ ont la même signification que dans la formule (I) et R' représente un groupement alkyle ($C_1$-$C_6$) linéaire ou ramifié,

composés de formule (I/a), (I/$a_1$), (I/b), (I/$b_1$), (I/c), (I/$c_1$) qui constituent l'ensemble des composés de formule (I),

- qui, lorsque $R_2$ représente un noyau phényle substitué par un groupement alkoxy ($C_1$-$C_6$) linéaire ou ramifié, peuvent être transformés, si on le désire, en composés de formule (I) dans lesquels $R_2$ représente un noyau phényle substitué par un groupement hydroxy,
- que l'on purifie, le cas échéant, selon une technique classique de purification,
- dont on sépare éventuellement les isomères selon une technique classique de séparation,
- et que l'on transforme, si on le souhaite, en leurs sels d'addition à un acide ou à une base pharmaceutiquement acceptable.

Les composés de formule (I) possèdent des propriétés pharmacologiques très intéressantes. En particulier, ils sont capables d'inhiber l'agrégation plaquétaire induite par le U46619 (9,11-didéoxy-11$\alpha$,9$\alpha$-époxyméthanoprostaglandine $F_{2\alpha}$), agoniste des récepteurs $TXA_2$, d'inhiber les contrations provoquées par le U46619 sur la trachée de cobaye et de prévenir in vivo les bronchoconstrictions induites par le U46619 chez le cobaye. En plus, les composés inhibent

la synthèse de $TXA_2$ dans le sang du lapin.

La présente invention a également pour objet les compositions pharmaceutiques renfermant comme principe actif au moins un composé de formule (I) seul ou en combinaison avec un ou plusieurs excipients ou véhicules inertes non toxiques.

Parmi les compositions pharmaceutiques selon l'invention on pourra citer plus particulièrement celles qui conviennent pour l'administration orale, parentérale, nasale, les comprimés simples ou dragéifiés, les comprimés sublinguaux, les gélules, les tablettes, les suppositoires, les crèmes, pommades, gels dermiques, etc...

La posologie utile varie selon l'âge et le poids du patient, la nature et la sévérité de l'affection ainsi que la voie d'administration. Celle-ci peut être orale, nasale, rectale ou parentérale, D'une manière générale, la posologie unitaire s'échelonne entre 10 et 200 mg pour un traitement en 1 à 3 prises par 24 heures.

Les exemples suivants illustrent l'invention et ne la limitent en aucune façon.

Les produits de départ utilisés sont des produits connus ou préparés selon des modes opératoires connus.

Les lettres α et β signifient que les hydrogènes de la pyrrolidine sont en cis l'un par rapport à l'autre dans le cas de (2α,3α) et en trans l'un par rapport à l'autre dans le cas de (2α,3β).

**<u>EXEMPLE 1</u> : Acide (4Z)-6-[(2α,3α)-1-(4-fluorophénylsulfonyl)-2-(3-méthoxy phényl) pyrrolidin-3-yl] hex-4-ènoïque, sel de sodium**

<u>STADE A</u> : [(2α,3α)-1-(4-fluorophénylsulfonyl)-2-(3-méthoxy phényl) pyrrolidin-3-yl] carboxylate d'éthyle

A une solution sous agitation, à température ambiante, contenant 77,4 mmoles de [(2α,3α)-2-(3-méthoxyphényl) pyrrolidin-3-yl] carboxylate d'éthyle (préparé selon le mode opératoire décrit dans Can. J. Chem., 60 (7), 926, 1982) et 92,9 mmoles de triéthylamine dans 300 ml de chloroforme, est ajoutée, par petites fractions, une solution contenant 85,2 mmoles de chlorure de l'acide 4-fluorobenzènesulfonique dans 100 ml de chloroforme. L'agitation est maintenue trois heures. Après lavage de la phase chloroformique par de l'eau, séchage et évaporation du solvant, le produit attendu est obtenu par filtration du résidu sur de la silice avec du dichlorométhane.
<u>Rendement</u> : 93 %
<u>Résonance magnétique nucléaire du proton</u> ($CDCl_3$/TMS) :
La constante de couplage J entre les deux protons géminés 2α et 3α de la pyrrolidine est égale à 8 Hz.

<u>STADE B</u> : (2α,3α)-1-(4-fluorophénylsulfonyl)-2-(3-méthoxy phényl)-3-(hydroxyméthyl) pyrrolidine

A une solution, sous agitation, à -50°C, sous atmosphère inerte, contenant 33,1 mmoles du composé obtenu au stade précédent dans 250 ml de toluène, est ajoutée, par petites fractions, une solution 1,5 M contenant 66,2 mmoles d'hydrure de diisobutylaluminium dans du toluène. L'agitation est maintenue 30 minutes en laissant revenir la température à l'ambiante. Le milieu réactionnel est alors hydrolysé à l'aide d'une solution à 50 % de bisulfite de sodium. Le produit attendu est obtenu après filtration, séchage et évaporation du filtrat et cristallise lentement.
<u>Rendement</u> : 80 %
<u>Point de fusion</u> : 71°C
<u>Résonance magnétique nucléaire du proton</u> ($CDCl_3$/TMS) :
La constante de couplage J entre les deux protons géminés 2α et 3α de la pyrrolidine est égale à 8 Hz.

<u>STADE C</u> : (2α,3α)-1-(4-fluorophénylsulfonyl)-2-(3-méthoxyphényl)-3-(cyanométhyl) pyrrolidine

Une solution contenant 31,7 mmoles du composé obtenu au stade précédent et 36,8 mmoles de chlorure de tosyle dans 175 ml de pyrridine est agitée 48 heures à température ambiante. Le milieu réactionnel est alors hydrolysé sur 400 g de glace et extrait plusieurs fois par du dichlorométhane. Les phases organiques sont séchées, évaporées et conduisent au tosylate correspondant qui est chauffé à 100°C pendant 18 heures dans 130 ml de diméthylsulfoxide en présence de 27 mmoles de cyanure de potassium. Après refroidissement et hydrolyse du milieu sur 400 g de glace, le produit attendu précipite, est filtré, lavé par de l'éther diisopropylique et séché.
<u>Rendement</u> : 60 %
<u>Point de fusion</u> : 134°C
<u>Résonance magnétique nucléaire du proton</u> ($CDCl_3$/TMS) :
La constante de couplage J entre les deux protons géminés 2α et 3α de la pyrrolidine est égale à 7,5 Hz.

<u>STADE D</u> : [(2α,3α)-1-(4-fluorophénylsulfonyl)-2-(3-méthoxyphényl) pyrrolidin-3-yl] acétaldéhyde

A une suspension, sous agitation, sous atmosphère inerte, à -60°C, contenant 21,4 mmoles du composé obtenu

au stade précédent dans 300 ml de toluène, est ajoutée, par petites fractions, une solution 1,5 M contenant 21,4 mmoles d'hydrure de diisobutylaluminium dans du toluène. L'ensemble est agité à -60°C puis 2 heures à -10°C. Le milieu réactionnel est alors hydrolysé à -10°C par 100 ml d'une solution méthanol/eau (50/50). Après filtration, le filtrat est extrait par du dichlorométhane. Les phases organiques sont alors séchées, évaporées et le produit attendu est obtenu après purification du résidu par chromatographie sur gel de silice en utilisant comme solvant d'élution un mélange toluène/méthanol (95/5).
Rendement : 60 %
Résonance magnétique nucléaire du proton ($CDCl_3$/TMS) :
La constante de couplage J entre les deux protons géminés 2α et 3α de la pyrrolidine est égale à 7,5 Hz.

STADE E : Acide (4Z)-6-[(2α,3α)-1-(4-fluorophénylsulfonyl)-2-(3-méthoxyphényl) pyrrolidin-3-yl] hex-4-ènoïque

A une suspension, sous agitation, à température ambiante, sous atmosphère inerte, contenant 10,6 mmoles de chlorure de l'acide 4-(triphénylphosphonio)butanoïque dans 40 ml de tétrahydrofurane anhydre, est ajoutée lentement 21,2 ml d'une solution 1N de terbutylate de potassium dans le tétrahydrofurane. Le milieu est agité une heure à température ambiante puis refroidi à 0°C avant l'addition, goutte à goutte, de 5,3 mmoles du composé obtenu au stade précédent en solution dans 5 ml de tétrahydrofurane. La réaction est suivie par chromatographie sur couche mince. Le milieu réactionnel est alors hydrolysé par 10 ml d'une solution saturée de chlorure d'ammonium. Après filtration, le filtrat est lavé par de l'éther. La phase aqueuse est acidifiée par de l'acide chlorhydrique et extraite par du dichlorométhane. Les phases organiques sont lavées par de l'eau, séchées et évaporées. Le produit attendu est obtenu sous forme acide après purification du résidu par chromatographie sur gel de silice en utilisant comme solvant d'élution un mélange chloroforme/méthanol (97/3).
Rendement : 50 %
Résonance magnétique nucléaire du Proton ($CDCl_3$/TMS) :
La constante de couplage J entre les deux protons géminés 2α et 3α de la pyrrolidine est égale à 7,5 Hz.

L'acide est alors transformé en sel de sodium correspondant par addition de soude 1N à une solution méthanolique de l'acide, agitation pendant une heure à température ambiante, évaporation et précipitation du sel à l'aide d'éther.

| Microanalyse élémentaire (sel de sodium) : | | | | |
|---|---|---|---|---|
| | C % | H % | N % | S% |
| calculé | 58,84 | 5,37 | 2,98 | 6,83 |
| trouvé | 59,09 | 5,39 | 3,03 | 6,61 |

Les exemples 2 à 6 ont été obtenus selon le même mode opératoire que celui décrit pour l'exemple 1 en utilisant les produits de départ appropriés.

**EXEMPLE 2 : Acide (4Z)-6-[(2α,3α)-1-(4-fluorophénylsulfonyl)-2-(pyridin-3-yl) pyrrolidin-3-yl]hex-4-ènoïque, sel de sodium**

Rendement (stade E) : 40 %
Résonance magnétique nucléaire du proton (DMSO $d_6$/TMS) :
La constante de couplage J entre les deux protons géminés 2α et 3α de la pyrrolidine est égale à 7,5 Hz.

| Microanalyse élémentaire : | | | | |
|---|---|---|---|---|
| | C % | H % | N % | S% |
| calculé | 57,26 | 5,03 | 6,36 | 7,28 |
| trouvé | 57,52 | 5,23 | 6,35 | 7,53 |

**EXEMPLE 3 : Acide (4z)-6-[(2α,3α)-1(4-fluorophénylsulfonyl)-2-(4-chlorophényl) pyrrolidin-3-yl] hex-4-ènoïque, sel de sodium**

Rendement (stade E) : 62 %
Résonance magnétique nucléaire du proton ($CDCl_3$/TMS) :
La constante de couplage J entre les deux protons géminés 2α et 3α de la pyrrolidine est égale à 7,5 Hz.
Spectre de masse : Impact électronique

$M^+$ : m/z = 451 (théorie : 451,9)

**<u>EXEMPLE 4 :</u> Acide (4Z)-6-[(2α,3α)-1-(4-fluorophénylsulfonyl)-2-phénylpyrrolidin-3-yl] hex-4-ènoïque, sel de sodium**

<u>Rendement</u> (stade E) : 45 %
<u>Résonance magnétique nucléaire du proton</u> ($CDCl_3$/TMS) :
La constante de couplage J entre les deux protons géminés 2α et 3α de la pyrrolidine est égale à 8 Hz.

| Microanalyse élémentaire : | | | | | |
|---|---|---|---|---|---|
| | C % | H % | N % | Cl % | S% |
| calculé | 57,96 | 5,08 | 3,07 | 7,78 | 7,03 |
| trouvé | 57,64 | 5,21 | 3,07 | 7,54 | 6,93 |

**<u>EXEMPLE 5 :</u> Acide(4Z)-6-[(2α,3α)-1-benzyl-2-(pyridin-3-yl) pyrrolidin-3-yl] hex-4-ènoïque, sel de sodium**

<u>Rendement</u> (stade E) : 30 %
<u>Résonance magnétique nucléaire du proton</u> ($CDCl_3$/TMS) :
La constante de couplage J entre les deux protons géminés 2α et 3α de la pyrrolidine est égale à 7,5 Hz.

| Microanalyse élémentaire : | | | |
|---|---|---|---|
| | C % | H % | N % |
| trouvé | 75,28 | 7,40 | 7,81 |
| calculé | 75,40 | 7,48 | 7,99 |

**<u>EXEMPLE 6 :</u> Acide (5Z)-7-[(2α,3α)-1-(4-fluorophénylsulfonyl)-2-(3-méthoxy phényl) pyrrolidin-3-yl] hept-5-ènoïque, sel de sodium**

<u>Rendement</u> (stade E) : 50 %
<u>Résonance magnétique nucléaire du proton</u> ($CDCl_3$/TMS) :
La constante de couplage J entre les deux protons géminés 2α et 3α de la pyrrolidine est égale à 7,5 Hz.

**<u>EXEMPLE 7 :</u> Acide (4Z)-6-{(2α,3α)-1-[(pyridin-2-yl) méthyl]-2-phénylpyrrolidin-3-yl) hex-4-ènoïque, sel de sodium**

<u>STADE A</u> : (2α,3α)-2-phényl-3-hydroxyméthyl pyrrolidine

A une suspension, à 0°C, de 68,4 mmoles d'hydrure de lithium aluminium dans 100 ml d'éther, est ajoutée, goutte à goutte, une solution contenant 68,4 mmoles de (2α,3α)-(2-phényl-pyrrolidin-3-yl)carboxylate d'éthyle (préparé selon le mode opératoire décrit dans Can. J. Chem., 60 (7), 926, 1982) dans 100 ml d'éther. Le milieu réactionnel est maintenu sous agitation 30 minutes à 0°C. On ajoute alors 15 ml d'acétate d'éthyle et 20 ml d'une solution aqueuse saturée en chlorure d'ammonium. Après filtration du précipité formé, le filtrat est lavé par de l'eau, et le produit attendu est obtenu après séchage et évaporation de la phase organique.
<u>Rendement :</u> 92 %
<u>Résonance magnétique nucléaire du proton</u> ($CDCl_3$/TMS) :
La constante de couplage J entre les deux protons géminés 2α et 3α de la pyrrolidine est égale à 7,3 Hz.

<u>STADE B</u> : (2α,3α)-1-[(pyridin-2-yl) méthyl]-2-phényl-3-hydroxy méthyl-pyrrolidine

Le produit attendu est obtenu, en procédant comme au stade A de l'exemple 1, mais en remplacant le chlorure de l'acide 4-fluorophénylsulfonique par la 2-chlorométhylpyridine.
<u>Rendement :</u> 87 %
<u>Résonance magnétique nucléaire du proton</u> ($CDCl_3$/TMS) :
La constante de couplage J entre les deux protons géminés 2α et 3α de la pyrrolidine est égale à 6 Hz.

STADES C à E : Les stades C à E de cet exemple sont identiques aux stades C à E de l'exemple 1.

Stade E

Rendement : 35 %
Résonance magnétique nucléaire du proton ($CDCl_3$/TMS) :
La constante de couplage J entre les deux protons géminés 2α et 3α de la pyrrolidine est égale à 8 Hz.

| Microanalyse élémentaire : | | | |
|---|---|---|---|
| | C % | H % | N % |
| calculé | 70,95 | 6,77 | 7,52 |
| trouvé | 71,07 | 6,98 | 7,47 |

**EXEMPLE 8 : Acide (4Z)-6-[(2α,3α)-1-nicotinoyl-2-phényl-pyrrolidin-3-yl]** hex-4-ènoïque, sel de sodium

Ce composé est synthétisé en utilisant le même mode opératoire que celui décrit dans l'exemple 7 en utilisant les produits de départ correspondants.

**EXEMPLE 9 : Acide (4Z)-6-[(2α-3α)-1-(4-fluorophénylsulfonyl)-2-(3-méthoxy phényl) pyrrolidin-3-yl] hex-4-ènoïque, sel de sodium**

STADE A : [(2α,3α)-1-(4-fluorophénylsulfonyl)-2-(3-méthoxyphényl) pyrrolidin-3-yl] carboxylate d'éthyle

Ce stade est identique au stade A de l'exemple 1.

STADE A' : [(2α,3β)-1-(4-fluorophénylsulfonyl)-2-(3-méthoxyphényl) pyrrolidin-3-yl] carboxylate d'éthyle

Le produit attendu est obtenu par épimérisation du composé décrit au stade précédent en agitant 40 mmoles de ce composé dans 30 ml d'éthanol, en présence de 40 mmoles d'éthylate de sodium, à température ambiante, pendant 2 heures. Après évaporation du solvant, le résidu est hydrolysé par 5 ml d'eau et extrait par du dichlorométhane. Le produit attendu est obtenu après séchage et évaporation des phases organiques.
Rendement : 90 %
Résonance magnétique nucléaire du proton ($CDCl_3$/TMS) :
La constante de couplage J entre les deux protons géminés 2α et 3β de la pyrrolidine est égale à 4,5 Hz.

STADES B à E : Ces stades sont identiques à ceux décrits dans l'exemple 1.

Rendement (stade E) : 38 %
Résonance magnétique nucléaire du proton ($CDCl_3$/TMS) :
La constante de couplage J entre les deux protons géminés 2α et 3β de la pyrrolidine est égale à 11 Hz.
Les exemples 10 et 11 ont été synthétisés selon le même procédé que celui décrit par l'exemple 9 en utilisant les produits de départ correspondants.

**EXEMPLE 10 : Acide (4Z)-6-[(2α,3β)-1-benzyl-2-(3-méthoxyphényl) pyrrolidin-3-yl] hex-4-ènoïque, chlorhydrate**

Rendement (stade E) : 53 %
Résonance magnétique nucléaire du proton ($CDCl_3$/TMS) :
La constante de couplage J entre les deux protons géminés 2α et 3β de la pyrrolidine est égale à 11 Hz.
Spectre de masse (acide) : Impact électronique
$M^+$ : m/z = 379 (théorie : 379,5)

**EXEMPLE 11 : Acide (5Z)-7-[(2α,3β)-1-benzyl-2-(3-méthoxyphényl) pyrrolidin-3-yl] hept-5-ènoïque, chlorhydrate**

Rendement (stade E) : 38 %
Résonance magnétique nucléaire du proton ($CDCl_3$/TMS) :

La constante de couplage J entre les deux protons géminés 2α et 3β de la pyrrolidine est égale à 11 Hz.

**EXEMPLE 12 : Acide (4Z)-5-[(2α,3α)-1-(4-chlorophénylsulfonyl)-2-phénylpyrrolidin-3-yl] pent-4-ènoïque, sel de sodium**

STADE A : [(2α,3α)-1-(4-chlorophénylsulfonyl-2-phényl-pyrrolidin-3-yl] carboxylate d'éthyle

Le produit attendu est obtenu en procédant comme au stade A de l'exemple 1 en utilisant le produit de départ correspondant.

STADE B : [(2α,3α)-1-(4-chlorophénylsulfonyl)-2-phényl-pyrrolidin-3-yl] acétaldéhyde

Le produit attendu est obtenu en procédant comme au stade D de l'exemple 1 en utilisant le produit décrit au stade précédent.

STADE C : Acide (4Z)-5-[(2α,3α)-1-(4-chlorophénylsulfonyl)-2-phényl-pyrrolidin-3-yl] pent-4-ènoïque, sel de sodium

Le produit attendu est obtenu en procédant comme au stade E de l'exemple 1 en utilisant le produit décrit au stade précédent.
Rendement : 20 %
Résonance magnétique nucléaire du proton ($CDCl_3$/TMS) :
La constante de couplage J entre les deux protons géminés 2α et 3α de la pyrrolidine est égale à 7 Hz.

**EXEMPLE 13 : Acide (5Z)-6-[(2α,3α)-1-(4-fluorophénylsulfonyl)-2-(4-chlorophényl) pyrrolidin-3-yl] hex-5-ènoïque**

Ce composé est obtenu selon le même procédé que celui décrit dans l'exemple 12 en utilisant les produits de départ correspondants.
Rendement (stade E) : 20 %
Résonance magnétique nucléaire du proton ($CDCl_3$/TMS) :
La constante de couplage J entre les deux protons géminés 2α et 3α de la pyrrolidine est égale à 7,5 Hz.

**EXEMPLE 14 : Acide (4Z)-6-[(2α,3α)-1-(4-fluorophénylsulfonyl)-2-(3-hydroxyphényl) pyrrolidin-3-yl] hex-4-ènoïque, sel de sodium**

A une solution, sous agitation à -78°C contenant 11 mmoles du composé décrit dans l'exemple 1 dans 50 ml de dichlorométhane est ajoutée, goutte à goutte, 0,84 ml d'une solution 8 M de tribromure de bore dans le dichlorométhane. Le milieu réactionnel est maintenu 4 heures sous agitation après retour à température ambiante. Il est alors hydrolysé par 10 ml d'une solution saturée en chlorure d'ammonium et 10 ml de méthanol. Après extraction par du dichlorométhane, les phases organiques sont séchées et évaporées. Le produit attendu est obtenu après purification du résidu par chromatographie sur gel de silice en utilisant comme solvant d'élution un mélange dichlorométhane/méthanol (95/5) et transformé en sel de sodium correspondant.
Rendement : 94 %
Résonance magnétique nucléaire du proton (DMSO $d_6$/TMS) :
La constante de couplage J entre les deux protons géminés 2α et 3α de la pyrrolidine est égale à 7,5 Hz.

| Microanalyse élémentaire : | | | | |
|---|---|---|---|---|
| | C % | H % | N % | S % |
| calculé | 58,01 | 5,09 | 3,08 | 7,07 |
| trouvé | 57,99 | 5,41 | 2,97 | 6,89 |

Les exemples 15, 16 et 17 ont été préparés selon le même procédé que celui décrit dans l'exemple 14 en utilisant les produits de départ appropriés.

**<u>EXEMPLE 15 :</u> Acide (5Z)-7-[(2α,3α)-1-(4-fluorophénylsulfonyl)-2-(3-hydroxyphényl) pyrrolidin-3-yl] hept-5-ènoïque, sel de sodium**

Rendement : 75 %
Résonance magnétique nucléaire du proton (DMSO $d_6$/TMS) :
La constante de couplage J entre les deux protons géminés 2α et 3α de la pyrrolidine est égale à 7,5 Hz.

| Microanalyse élémentaire : | | | | |
|---|---|---|---|---|
| | C % | H % | N % | S % |
| calculé | 58,84 | 5,37 | 2,98 | 6,83 |
| trouvé | 58,47 | 5,49 | 3,03 | 6,94 |

**<u>EXEMPLE 16 :</u> Acide (4Z)-6-[(2α,3β)-1-benzyl-2-(3-hydroxyphényl) pyrrolidin-3-yl] hex-4-ènoïque, bromhydrate**

Rendement : 80 %
Résonance magnétique nucléaire du proton (DMSO $d_6$/TMS) :
La constante de couplage J entre les deux protons géminés 2α et 3β de la pyrrolidine est égale à 10 Hz.

| Microanalyse élémentaire : | | | | |
|---|---|---|---|---|
| | C % | H % | N % | S % |
| calculé | 61,89 | 6,32 | 3,14 | 17,90 |
| trouvé | 61,83 | 6,40 | 3,02 | 17,60 |

**<u>EXEMPLE 17 :</u> Acide (4Z)-6-[(2α,3β)-1-(4-fluorophénylsulfonyl)-2-(3-hydroxyphényl) pyrrolidin-3-yl] hex-4-ènoïque, sel de sodium**

Rendement : 74 %
Résonance magnétique nucléaire du proton (acide) ($CDCl_3$/TMS) :
La constante de couplage J entre les deux protons géminés 2α et 3β de la pyrrolidine est égale à 11 Hz.

| Microanalyse élémentaire : | | | | |
|---|---|---|---|---|
| | C % | H % | N % | S % |
| calculé | 58,01 | 5,09 | 3,08 | 7,04 |
| trouvé | 57,91 | 5,39 | 3,09 | 6,87 |

**<u>EXEMPLE 18 :</u> Acide 7-[(2α,3α)-1-(4-fluorophénylsulfonyl)-2-(3-hydroxyphényl) pyrrolidin-3-yl] heptanoïque, sel de sodium**

Une solution contenant 1,06 mmoles du composé décrit dans l'exemple 15, dans 25 ml d'éthanol, est agitée, à température ambiante et pression atmosphérique sous atmosphère d'hydrogène, en présence de 50 mg de palladium sur charbon à 10 %. Après filtration du catalyseur, le produit attendu est obtenu par évaporation du solvant et cristallisation dans de l'éther.
Rendement : 99 %
Point de fusion : 180-182°C
Résonance magnétique nucléaire du proton ($CDCl_3$/TMS) :
La constante de couplage J entre les deux protons géminés 2α et 3α de la pyrrolidine est égale à 7 Hz.

| Microanalyse élémentaire : | | | | |
|---|---|---|---|---|
| | C % | H % | N % | S % |
| calculé | 58,59 | 5,77 | 2,97 | 6,80 |
| trouvé | 57,92 | 5,83 | 3,03 | 6,47 |

Les exemples 19 et 20 ont été préparés selon le même mode opératoire que celui décrit dans l'exemple 18 en utilisant les produits de départ appropriés.

**EXEMPLE 19 : Acide 6-[(2α,3α)-1-phénylsulfonyl-2-phényl-pyrrolidin-3-yl] hexanoïque, sel de sodium**

Rendement : 99 %
Point de fusion : 170-174°C
Résonance magnétique nucléaire du proton ($CDCl_3$/TMS) :
La constante de couplage J entre les deux protons géminés 2α et 3α de la pyrrolidine est égale à 7,5 Hz.

| Microanalyse élémentaire : | | | | |
|---|---|---|---|---|
| | C % | H % | N % | S % |
| calculé | 62,39 | 6,19 | 3,31 | 7,57 |
| trouvé | 62,21 | 6,21 | 3,41 | 6,79 |

**EXEMPLE 20 : Acide 6-[(2α,3α)-1-(4-fluorophénylsulfonyl)-2-(pyridin-3-yl) pyrrolidin-3-yl] hexanoïque, sel de sodium**

Rendement : 99 %
Résonance magnétique nucléaire du proton ($CDCl_3$/TMS) :
La constante de couplage J entre les deux protons géminés 2α et 3α de la pyrrolidine est égale à 7,5 Hz.

| Microanalyse élémentaire : | | | | |
|---|---|---|---|---|
| | C % | H % | N % | S % |
| calculé | 57,00 | 5,47 | 6,33 | 7,25 |
| trouvé | 56,92 | 5,55 | 6,20 | 6,81 |

**EXEMPLE 21 : Acide (4Z)-6-[(2α,3α)-1[(pyridin-3-yl)méthyl]-2-phénylpyrrolidin-3-yl]hex-4-ènoïque, sel de sodium**

STADE A : (2α,3α)-[1-(Pyridin-3-yl)méthyl-2-phénylpyrrolidin-3-yl] carboxylate d'éthyle

A une suspension agitée, à température ambiante, contenant 68,4 mmoles de (2α,3α)-(2-phénylpyrrolidin-3-yl) carboxylate d'éthyle (préparé selon le mode opératoire décrit dans Can. J. Chem. 60, (7), 926, 1982) et 167 mmoles de carbonate de potassium dans 200 ml d'acétonitrile, est ajoutée, par petites fractions, une solution de 75,2 mmoles de 3-chlorométhylpyridine dans 50 ml d'acétonitrile. L'agitation est maintenue 48 heures. Après filtration des sels et concentration du solvant, le produit est obtenu par chromatographie sur gel de silice en utilisant comme éluant un mélange dichlorométhane/méthanol (95/5).
Rendement : 93 %
Résonance magnétique nucléaire du proton ($CDCl_3$/TMS) :
La constante de couplage J entre les deux protons géminés 2α et 3α de la pyrrolidine est égale à 8 Hz.

STADE B : (2α,3α)-1-(Pyridin-3-yl)méthyl-2-phényl-3-hydroxyméthyl pyrrolidine

Le produit attendu est obtenu en procédant comme au stade A de l'exemple 7 mais en remplaçant l'éther par le tétrahydrofurane.
Rendement : 66 %
Point de fusion : 139°C
Résonance magnétique nucléaire du proton ($CDCl_3$/TMS) :
La constante de couplage J entre les deux protons géminés 2α et 3α de la pyrrolidine est égale à 7 Hz.

STADES C ET D : identiques aux stades C et D de l'exemple 1.

STADE E : (2α,3α)-[1-(Pyridin-3-yl)méthyl-2-phénylpyrrolidin-3-yl) hex-4-ènoate de méthyle

A une suspension, sous agitation, à température ambiante, sous atmosphère inerte, contenant 10,6 mmoles de chlorure de l'acide 4-triphénylphosphoniobutanoïque dans 40 ml de tétrahydrofurane anhydre, est ajoutée lentement 21,2 ml d'une solution 1N de terbutylate de potassium dans le tétrahydrofurane. Le milieu est agité une heure à température ambiante puis refroidi à 0°C avant l'addition goutte à goutte de 5,3 mmoles du composé obtenu au stade précédent en solution dans 5 ml de tétrahydrofurane. Le milieu réactionnel est agité 2 heures à température ambiante puis hydrolysé par 10 ml d'une solution saturée de chlorure d'ammonium. Après filtration, le filtrat est lavé par de l'éther. La phase aqueuse est ramenée à pH = 7 par addition d'acide acétique puis extraite par du dichlorométhane. La phase organique est refroidie à 0°C et une solution de diazométhane dans l'éther est ajoutée goutte à goutte jusqu'à persistance de la coloration jaune. L'excès de diazométhane est détruit par addition d'une goutte d'acide acétique. Le milieu réactionnel est séché sur sulfate de magnésium, le solvant est évaporé. Le produit attendu est obtenu après purification du résidu par chromatographie sur gel de silice en utilisant comme éluant le mélange dichlorométhane/méthanol.
Rendement : 93 %
Résonance magnétique nucléaire du proton ($CDCl_3$/TMS) :
La constante de couplage J entre les deux protons géminés 2α et 3α de la pyrrolidine est égale à 8 Hz.

STADE F : Acide (4Z)-6-[(2α,3α)-1[(pyridin-3-yl)méthyl]-2-phénylpyrrolidin-3-yl]hex-4-ènoïque, sel de sodium

Le produit obtenu au stade précédent en solution dans 50 ml de méthanol est saponifié par addition de 35 ml de soude 1N. Après un reflux de 2 heures, le milieu est concentré de moitié, le produit attendu est extrait au dichlorométhane.
Rendement : 46 %
Résonance magnétique nucléaire du proton ($CDCl_3$/TMS) :
La constante de couplage J entre les deux protons géminés 2α et 3α de la pyrrolidine est égale à 7 Hz.

| Microanalyse élémentaire : | | | |
|---|---|---|---|
| | C % | H % | N % |
| calculé | 70,95 | 6,77 | 7,52 |
| trouvé | 71,07 | 7,05 | 7,47 |

**EXEMPLE 22 : Acide 6-[(2α,3α)-1-[(pyridin-3-yl)méthyl]-2-phénylpyrrolidin-3-yl]hexanoïque, sel de sodium**

STADE A : 6-[2α,3α)-1-[(pyridin-3-yl)méthyl]-2-phénylpyrrolidin-3-yl]hexanoate de méthyle

Ce composé est préparé à partir du composé décrit au stade E de l'exemple 21 selon le même mode opératoire que celui décrit dans l'exemple 18.
Rendement : 99 %
Résonance magnétique nucléaire du proton ($CDCl_3$/TMS) :
La constante de couplage J entre les deux protons géminés 2α et 3α de la pyrrolidine est égale à 8 Hz.

STADE B : Acide 6-[(2α,3α)-1-[(pyridin-3-yl)méthyl]-2-phényl pyrrolidin-3-yl]hexanoïque, sel de sodium

Identique au stade F de l'exemple 21.
Rendement : 57 %
Point de fusion : 164°C

| Microanalyse élémentaire : | | | |
|---|---|---|---|
| | C % | H % | N % |
| calculé | 70,57 | 7,27 | 7,48 |
| trouvé | 69,69 | 7,12 | 7,44 |

**EXEMPLE 23 : Acide (4Z) [(2α,3α)-1-terbutoxycarbonyl-2-(pyridin-3-yl)-pyrrolidin-3-yl]hex-4-ènoïque, sel de sodium**

STADE A : (2α,3α)-terbutoxycarbonyl-2-(pyridin-3-yl)-3-hydroxyméthyl pyrrolidine

A une suspension à 0°C de 0,808 mole d'hydrure de lithium aluminium dans 500 ml d'éther, est ajoutée, goutte à goutte, une solution contenant 0,404 mole de (2α,3α)-[2-(3-pyridyl)pyrrolidin-3-yl]carboxylate d'éthyle (préparé selon Can. J. Chem. 60, (7), 926, 1982) dans 1 l d'éther. Le milieu réactionnel est maintenu sous agitation 1 heure à 0°C. On ajoute alors 50 ml d'acétate d'éthyle puis 200 ml d'eau. Après filtration du précipité formé et reprise par de la soude 0,1 N, l'éther est évaporé et la phase aqueuse résultante, après ajout de 200 ml de dioxane, est refroidie à 0°C. On ajoute une solution contenant 0,412 mole de carbonate de terbutyle dans 200 ml de dioxane. Le milieu réactionnel est agité une heure à température ambiante, extrait au dichlorométhane. Le produit attendu est cristallisé, après évaporation des solvants dans un mélange éther isopropylique/pentane (50/50).
Rendement : 69 %
Point de fusion : 90,5°C

STADES B à E : les stades B à E de cet exemple sont identiques aux stades C à F de l'exemple 21.

STADE F : Acide (4Z) [(2α,3α)-1-terbutoxycarbonyl-2-(pyridin-3-yl)-pyrrolidin-3-yl]hex-4-ènoïque, sel de sodium

Rendement : 51 %

| Microanalyse élémentaire : | | | |
|---|---|---|---|
| | C % | H % | N % |
| calculé | 62,81 | 7,12 | 7,32 |
| trouvé | 61,90 | 6,95 | 7,13 |

**EXEMPLE 24 : Acide (4Z)-6-[(2α,3α)-1-(4-chlorophénylaminocarbonyl)-2-(pyridin-3-yl)pyrrolidin-3-yl]hex-4-ènoïque, sel de sodium**

STADE A : (4Z)-6-[(2α,3α)-2-(pyridin-3-yl)pyrrolidin-3-yl]hex-4-ènoate de méthyle

18,4 mmoles du composé préparé au stade D de l'exemple 23 sont agitées dans 50 ml d'acide trifluoroacétique, à température ambiante, pendant 1/2 heure. Le milieu réactionnel est hydrolysé par 250 ml d'eau et extrait au dichlorométhane. La phase aqueuse est basifiée à pH = 10 par addition de soude 12N. Le produit attendu est extrait au dichlorométhane.
Rendement : 64 %

| Microanalyse élémentaire : | | | |
|---|---|---|---|
| | C % | H % | N % |
| calculé | 70,04 | 8,08 | 10,21 |
| trouvé | 69,46 | 8,05 | 10,07 |

STADE B : (4Z)-6-[(2α,3α)-1-(4-Chlorophénylaminocarbonyl)-2-(pyridin-3-yl)pyrrolidin-3-yl]hex-4-ènoate de méthyle

A une solution, sous agitation, à température ambiante, contenant 6,2 mmoles du composé préparé au stade précédent dans 20 ml de tétrahydrofurane, est ajoutée, par petites quantités, une solution contenant 6,2 mmoles d'isocyanate de 4-chlorophényl dans 20 ml de tétrahydrofurane. L'agitation est maintenue 48 heures. Après concentration du solvant, le produit attendu est purifié par chromatographie sur gel de silice en utilisant comme éluant le mélange dichlorométhane/méthanol (95/5).
Rendement : 57 %

STADE C : Acide (4Z)-6-[(2α,3α)-1-(4-chlorophénylaminocarbonyl)-2-(pyridin-3-yl)pyrrolidin-3-yl]hex-4-ènoïque, sel de sodium

Ce stade est identique au stade F de l'exemple 21.
Rendement : 73 %

| Microanalyse élémentaire : | | | | |
|---|---|---|---|---|
| | C % | H % | N % | Cl % |
| calculé | 60,62 | 5,32 | 9,64 | 8,13 |
| trouvé | 59,21 | 5,33 | 9,28 | 8,22 |

**EXEMPLE 25 : Acide (4Z)-6-[(2α,3α)-1-(2-hydroxybenzoyl)-2-(pyridin-3-yl) pyrrolidin-3-yl]hex-4-ènoïque, sel de sodium**

STADE A : (4Z)-6-[(2α,3α)-1-(2-Méthoxybenzoyl)-2-(pyridin-3-yl) pyrrolidin-3-yl]hex-4-ènoate de méthyle

A une solution agitée à température ambiante de 5,5 mmoles du composé préparé au stade A de l'exemple 24 et de 7,2 mmoles de triéthylamine dans 50 ml de toluène est ajoutée, par petites fractions, une solution contenant 6,6 mmoles de chlorure de l'acide o-méthoxybenzoïque dans 20 ml de toluène. Le milieu réactionnel est agité 2 heures à température ambiante puis traité à l'eau. Le produit attendu est obtenu après séchage et évaporation du solvant.
Rendement : 85 %
Résonance magnétique nucléaire du proton ($CDCl_3$/TMS) :
La constante de couplage J entre les deux protons géminés 2α et 3α de la pyrrolidine est égale à 7 Hz.

STADE B : (4Z)-6-[(2α,3α)-1-(2-hydroxybenzoyl)-2-(pyridin-3-yl) pyrrolidin-3-yl]hex-4-ènoate de méthyle

Le produit attendu est obtenu à partir du composé décrit au stade précédent en utilisant le procédé décrit dans l'exemple 14.
Rendement : 35 %
Point de fusion : 77°C

STADE C : Acide (4Z)-6-[(2α,3α)-1-(2-hydroxybenzoyl)-2-(pyridin-3-yl) pyrrolidin-3-yl]hex-4-ènoïque, sel de sodium

Identique au stade F de l'exemple 21.
Rendement : 70 %

| Microanalyse élémentaire : | | | |
|---|---|---|---|
| | C % | H % | N % |
| calculé | 65,66 | 5,76 | 6,96 |
| trouvé | 64,78 | 5,40 | 6,90 |

**EXEMPLE 26 : Acide (4Z)-6-[(2α,3α)-1-(pyridin-3-yl)méthyl-2-(3-hydrozyphényl)pyrrolidin-3-yl]hex-4-ènoïque, sel de sodium**

STADE A : (2α,3α)-1-Terbutoxycarbonyl-2-(3-méthoxyphényl)-3-hydroxy méthyl pyrrolidine

Préparé selon le procédé décrit dans le stade A de l'exemple 23.
Rendement : 60 %
Résonance magnétique nucléaire du proton ($CDCl_3$/TMS) :
La constante de couplage J entre les deux protons géminés 2α et 3α de la pyrrolidine est égale à 9 Hz.

STADE B à E : Les stades B à D de cet exemple sont identiques aux stades C à E de l'exemple 21, le stade E est identique au stade A de l'exemple 24.

STADE F : (4Z)-6-[(2α,3α)-1-(pyridin-3-yl)méthyl-2-(3-méthoxyphényl) pyrrolidin-3-yl]hex-4-ènoate de méthyle

Ce produit est préparé selon le même procédé que celui décrit dans le stade A de l'exemple 21 en utilisant comme produit de départ le produit préparé au stade précédent.
Rendement : 70 %
Résonance magnétique nucléaire du proton ($CDCl_3$/TMS) :
La constante de couplage J entre les deux protons géminés 2α et 3α de la pyrrolidine est égale à 7 Hz.

STADE G : (4Z)-6-[(2α,3α)-1-(pyridin-3-yl)méthyl-2-(3-hydroxyphényl pyrrolidin-3-yl]hex-4-ènoate de méthyle

Préparé selon le même procédé que celui décrit dans l'exemple 14.
Rendement : 99 %

STADE H : Acide (4Z)-6-[(2α,3α)-1-(pyridin-3-yl)méthyl-2-(3-hydroxyphényl)pyrrolidin-3-yl]hex-4-ènoïque, sel de sodium

Préparé selon le même procédé que celui décrit au stade F de l'exemple 21.
Rendement : 40 %

| Microanalyse élémentaire : | | | |
|---|---|---|---|
| | C % | H % | N % |
| calculé | 72,11 | 7,15 | 7,64 |
| trouvé | 71,53 | 7,14 | 7,92 |

ETUDE PHARMACOLOGIQUE DES DERIVES DE L'INVENTION

**EXEMPLE 27 : Agrégation plaquettaire**

Les lapins (2-3 kg) sont anesthésiés avec du sodium pentobarbital (30 mg/kg i.v.). Après cannulation de l'artère carotide gauche, le sang est prélevé sur citrate de sodium (0.109 M) (1 vol. de citrate pour 9 vol. de sang).

Le plasma riche en plaquettes (PRP) est obtenu par centrifugation (20°C) à 250 g pendant 20 minutes et le plasma pauvre en plaquettes (PPP) par centrifugation à 1000 g (10 min). Le nombre des plaquettes (PL) dans le PRP est ajusté entre 300-350000 pl/$mm^3$ par dilution avec du PPP autologue. Le PRP est conservé à la température de la pièce jusqu'au moment du test et est utilisé dans les 4 heures qui suivent le prélèvement.

L'agrégation plaquettaire est réalisée à 37°C dans des tubes en verre siliconés à l'aide d'un agrégomètre. Le PRP et les PL sont agités à 1000 rpm (révolution par minute). Afin d'étudier l'activité des antagonistes du thromboxane, le PRP est incubé 1 min à 37°C, puis l'antagoniste est ajouté pour une durée de 3 min avant l'addition de l'agoniste U46619 (1.2 μM). Le volume final de la cuve est alors de 250 μl. L'intensité de l'agrégation plaquettaire est établie en prenant l'amplitude maximale de tracés agrégants et est exprimée en pourcentage de transmission lumineuse (% T). L'activité des antagonistes est exprimée en $IC_{50}$, c'est-à-dire la concentration de la substance qui induit 50 % d'inhibition de la réponse agrégante induite par le U46619.

Lors de ce test, les $IC_{50}$ des composés des exemples 14 et 15 sont égales à $2.10^{-5}$M.

**EXEMPLE 28 : Détermination des valeurs des $pA_2$ sur la trachée de cobaye**

Des cobayes mâles albinos de 400-500 grammes ont été sacrifiés par un coup sur la nuque et par élongation cervicale. La gorge est ouverte et la trachée est rapidement prélevée et puis découpée en anneaux de deux cartilages. Ces anneaux sont montés entre deux crochets dans des cuves thermostatées à 37°C contenant du liquide physiologique (composition en mM:NaCl 118 ; $NaHCO_3$ 25 ; Glucose 10 ; KCl 4.7 ; $CaCl_2$ 1.25 ; $MgSO_4$ 1.19 ; $KH_2PO_4$ 1.14).

La solution physiologique est bullée par un mélange de 95 % $O_2$ - 5 % $CO_2$. Le crochet inférieur constitue le point fixe tandis que le crochet supérieur est relié à un capteur de force isométrique. Les tissus sont mis sous une tension de base de 3,5 grammes. Les substances pharmacologiques étudiées sont préparées immédiatement avant l'usage. Les drogues sont solubilisées dans l'eau ou dans le diméthylsulfoxyde.

Après le montage, les préparations sont laissées en repos pendant 90 minutes, des rinçages étant effectués toutes les 30 minutes. Après réajustement de la tension de base, une contration provoquée par une dose unique d'agoniste (U46619 ; $10^{-5}$M) est provoquée afin de régulariser les contractions suivantes. Après lavage et retour à la ligne de base, une première courbe effet/concentration est réalisé par adjonction de doses cumulatives d'U46619 ($10^{-9}$M à $10^{-5}$M (l'espacement entre les doses est d'un demi-log). Cette première expérience permet de calculer la concentration efficace 50 % ($EC_{50}$) "témoin".

Cette $EC_{50}$ est calculée en routine de la manière suivante : les valeurs de tension sont d'abord converties en pourcentages par rapport à l'effet maximum, ces pourcentages sont ensuite reportés sur un graphique avec les pourcentages en ordonnée et les log (concentration) en abscisse. Une régression linéaire est alors effectuée sur les points compris entre 10 % et 90 % (ce qui correspond à la partie linéaire de la courbe sigmoïde). La concentration correspondant au demi effet maximum (50 %) peut être facilement calculée à l'aide des paramètres de la droite.

Après lavage et retour à la ligne de base, l'organe est mis en contact avec l'antagoniste (8 concentrations différentes pour chaque organe) pendant 20 minutes. Une deuxième courbe effet/concentration est alors réalisée en présence de l'antagoniste et l'$EC_{50}$ "traité" peut alors être calculée. On dispose ainsi de tous les éléments permettant le calcul du $pA_2$ (antagonisme compétitif) ou $pD_2$ (antagonisme non compétitif).

Le $pA_2$ (qui représente le logarithme négatif de la concentration d'antagoniste en présence de laquelle il faut 2 fois plus d'agoniste pour obtenir le même effet) est déterminé en reportant sur un graphe les valeurs de log ((L/l)-1) par rapport à log (concentration d'antagoniste) avec L = effet en présence d'antagoniste et 1 = effet témoin.

Lors de ce test, les valeurs des $pA_2$ des composés des exemples 1, 14 et 15 sont les suivantes :

| | |
|---|---|
| Exemple 1 : $pA_2$ = 7,2 | Exemple 21 : $pA_2$ = 8,74 |
| Exemple 14 : $pA_2$ = 8 | Exemple 22 : $pA_2$ = 5,15 |
| Exemple 15 : $pA_2$ = 7,4 | Exemple 26 : $pA_2$ = 7,51 |

**<u>EXEMPLE 29</u> : $IC_{50}$ sur la pression trachéale chez le cobaye**

Des cobayes albinos mâles (350-400 g) soumis à une diète hydrique de 18 heures sont anesthésiés au carbamate d'éthyle (1.25 g/kg i.p.). Un cathéter est introduit dans l'artère carotide afin de mesurer la pression artérielle au moyen d'une cellule de pression. Un deuxième cathéter est introduit dans la veine jugulaire et sert à injecter les substances pharmacologiques. La trachée est cannulée et le cobaye est mis en respiration assitée au moyen d'un respirateur. La température de l'animal est maintenue à 37°C à l'aide d'une couverture thermostatée. Une aiguille piquée dans la cannule trachéale est reliée à une cellule de pression et permet d'enregistrer la pression trachéale.

Les cobayes sont prétraités par la d-tubocurarine (1 mg/kg i.v.) et par l'indométhacine (10 mg/kg i.v.). Injectée à la dose de 2 µg/kg i.v., le U46619 provoque une bronchoconstriction qui entraine une augmentation de la pression trachéale et induit une augmentation de la pression artérielle. Les réponses au U46619 sont réversibles et reproductibles si les injections sont effectuées toutes les 10 minutes.

Les antagonistes des récepteurs au thromboxane sont injectés 5 minutes avant les injections d'U46619. La dose d'antagoniste inhibant de 50 % l'augmentation de la pression trachéale causée par le U46619 est recherchée ($IC_{50}$).

| | |
|---|---|
| Exemple 4 : | $IC_{50}$ = 0,7 mg/kg |
| Exemple 14 : | $IC_{50}$ = 0,07 mg/kg |
| Exemple 15 : | $IC_{50}$ = 0,16 mg/kg |
| Exemple 21 : | $IC_{50}$ = 0,17 mg/kg |
| Exemple 26 : | $IC_{50}$ = 0,03 mg/kg |

**<u>EXEMPLE 30</u> : Activité anti-thromboxane-synthase**

Des lapins néo-zélandais albinos d'environ 2 kg sont utilisés. Ces lapins sont anesthésiés au pentobarbital (30 mg/kg, I.V.). Le sang est recueilli par un cathéter carotidien dans des tubes secs en verre, à raison de 1 ml par tube, en présence ou absence de différentes concentrations d'inhibiteur. Les inhibiteurs sont dilués dans du serum physiologique afin d'éviter une hémolyse éventuelle.

Les tubes sont placés dans un bain-marie à 37°C pendant une heure. Après coagulation, les tubes sont refroidis à 4°C et centrifugés à 2000 g pendant 20 minutes afin de séparer le serum du caillot. Le serum peut être congelé à -20°C pendant 2 semaines pour un dosage ultérieur.

Les taux de $TXB_2$, le métabolite stable de $TXA_2$, du serum sont déterminés par Radio-Immunoassay.

Les taux obtenus en présence des différentes doses d'inhibiteur sont transformés en pourcentage d'inhibition par

rapport au taux basal de $TXB_2$, afin de calculer l'$IC_{50}$ de l'inhibiteur. Celle-ci est obtenue par régression non linéaire à l'aide de l'équation suivante (Michaelis L, Menten ML, Die Kinetik der invertinwirkung, Biochem. Zeitschrifft, 1913, 49, 333-369) :

$$I = (Imax * C^n) / (IC^n + C^n)$$

avec

| | |
|---|---|
| I = | inhibition |
| Imax = | inhibition maximum |
| C = | concentration d'inhibiteur |
| IC = | $IC_{50}$ |
| n = | coefficient de Hill |

| | |
|---|---|
| Exemple 2 : | $IC_{50} = 2{,}10^{-4}M$ |
| Exemple 20 : | $IC_{50} = 6.10^{-5}M$ |
| Exemple 21 : | $IC50 = 8{,}5.10^{-6}M$ |

**EXEMPLE 31 : Composition pharmaceutique**

Formule de préparation pour 1000 comprimés dosés à 10 mg

| | |
|---|---|
| Composé de l'exemple 1 | 10 g |
| Hydroxypropylcellulose | 2 g |
| Amidon de blé | 10 g |
| Lactose | 100 g |
| Stéarate de magnésium | 3 g |
| Talc | 3 g |

**Revendications**

**Revendications pour les Etats contractants suivants : AT, BE, CH, DE, DK, FR, GB, IE, IT, LI, LU, NL, PT, SE**

**1.** Composés de formulé (I) :

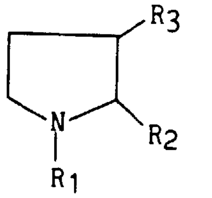

(I)

dans laquelle :
$R_1$ représente :

- un groupement alkyl ($C_1$-$C_6$) linéaire ou ramifié substitué ou non par un groupement pyridin-2-yl, pyridin-3-yl, phényl (lui-même éventuellement substitué par un ou plusieurs atomes d'halogène ou groupements alkyle ($C_1$-$C_6$) linéaire ou ramifié, alkoxy ($C_1$-$C_6$) linéaire ou ramifié, trihalogénométhyle),
- un groupement phényle substitué ou non par un ou plusieurs atomes d'halogène ou groupements alkyle ($C_1$-$C_6$) linéaire ou ramifié, alkoxy ($C_1$-$C_6$) linéaire ou ramifié, trihalogénométhyle,
- un groupement pyridyle,
- un groupement phénylsulfonyl substitué ou non sur le noyau phényle par un ou plusieurs atomes d'halogène ou groupements alkyle ($C_1$-$C_6$) linéaire ou ramifié, alkoxy ($C_1$-$C_6$) linéaire ou ramifié, trihalogénométhyle,

- un groupement acyle ($C_1$-$C_6$) linéaire ou ramifié,

- un groupement alkoxycarbonyl ($C_1$-$C_6$) linéaire ou ramifié,

- un groupement benzoyle (substitué ou non sur le noyau phényl par un ou plusieurs atomes d'halogène ou groupements hydroxy, alkyle, alkoxy, trifluorométhyle) ou pyridylcarbonyl,

- un groupement alkylaminocarbonyle ou phénylaminocarbonyle (substitué ou non sur le noyau phényl par un ou plusieurs atomes d'halogène ou groupements hydroxy, alkyle, alkoxy, trifluorométhyle),

- un groupement acylamino ou benzoylamino,

$R_2$ représente :

- un groupement phényle substitué ou non par un ou plusieurs atomes d'halogène ou groupements alkyle ($C_1$-$C_6$) linéaire ou ramifié, alkoxy ($C_1$-$C_6$) linéaire ou ramifié, hydroxy, trihalogénométhyle,

- un groupement pyridin-3-yle ou pyridin-2-yle,

$R_3$ représente l'un quelconque des groupements suivants :

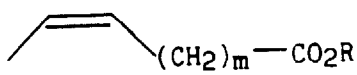

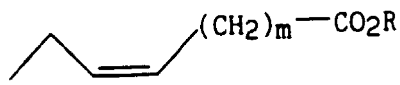

$-(CH_2)_n-CO_2R$

dans lesquels

m est égal à 2, 3 ou 4,

n est égal à 4, 5, 6 ou 7,

et R représente un atome d'hydrogène ou un groupement alkyle ($C_1$-$C_6$) linéaire ou ramifié,

leurs énantiomères, diastéréoisomères et épimères ainsi que leurs sels d'addition à un acide ou à une base pharmaceutiquement acceptable.

2. Composés de formule (I) selon la revendication 1 dans laquelle $R_1$ représente un groupement phénylsulfonyl substitué ou non, leurs énantiomères, diastéréoisomères et épimères ainsi que leurs sels d'addition à un acide ou à une base pharmaceutiquement acceptable.

3. Composés de formule (I) selon la revendication 1 dans laquelle $R_1$ représente un groupement pyridin-3-yl-méthyl, leurs énantiomères, diastéréoisomères et épimères ainsi que leurs sels d'addition à un acide ou à une base pharmaceutiquement acceptable.

4. Composés de formule (I) selon la revendication 1 dans laquelle $R_2$ représente un groupement phényle substitué ou non, leurs énantiomères, diastéréoisomères et épimères ainsi que leurs sels d'addition à un acide ou à une base pharmaceutiquement acceptable.

5. Composé de formule (I) selon l'une quelconque des revendications 1, 3 ou 4 qui est l'acide (4Z)-6-[(2α,3α)-1-[(pyridin-3-yl)méthyl]-2-phénylpyrrolidin-3-yl]hex-4-ènoïque, ses énantiomères, diastéréoisomères ainsi que ses sels d'addition à une base ou un acide pharmaceutiquement acceptable.

**6.** Procédé de préparation des dérivés de formule (I) selon la revendication 1 caractérisé en ce que l'on utilise comme produit de départ une pyrrolidine de formule (II) :

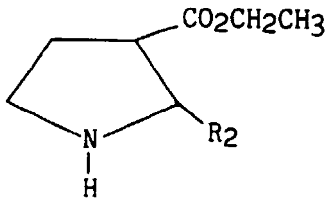

(II)

dans laquelle $R_2$ a la même signification que dans la formule (I), sous forme racémique ou dont on a préalablement séparé les isomères selon une technique classique de séparation,
que l'on fait réagir :

- soit avec de l'hydrure de lithium aluminium dans de l'éther, pour conduire à la pyrrolidine de formule (III) :

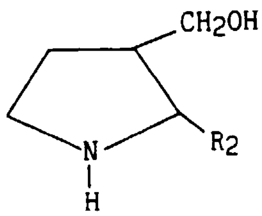

(III)

dans laquelle $R_2$ a la même signification que dans la formule (I),
que l'on met alors en réaction avec un dérivé halogéné de formule $R_1X$ dans laquelle $R_1$ a la même signification que dans la formule (I) et X représente un atome d'halogène, dans du chloroforme en présence de triéthylamine ou dans de l'acétonitrile en présence de carbonate de potassium,
pour conduire à la pyrrolidine de formule (IV) :

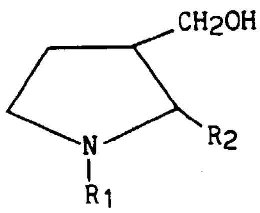

(IV)

dans laquelle $R_1$ et $R_2$ ont la même signification que dans la formule (I),

- soit avec un dérivé halogéné de formule $R_1X$ tel que défini précédemment,
pour conduire à la pyrrolidine de formule (V) :

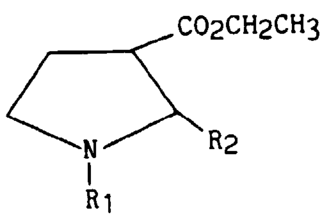

(V)

dans laquelle $R_1$ et $R_2$ ont la même signification que dans la formule (I),
composé de formule (V) qui, lorsqu'il est sous forme d'un couple d'énantiomères, peut être transformé, si on le souhaite, en son autre couple d'énantiomères par réaction de l'éthanolate de sodium, en milieu éthanolique, composé de formule (V) que l'on réduit :

a ou bien :

en présence de deux équivalents d'hydrure de diisobutylaluminium dans du toluène,

pour conduire à la pyrrolidine de formule (IV) définie précédemment, composé de formule (IV), qui, quelle que soit la voie de synthèse par lequel il a été obtenu, est transformé en tosylate (Ts) correspondant de formule (VI), par réaction avec du chlorure de toluène sulfonyle en présence de pyridine,

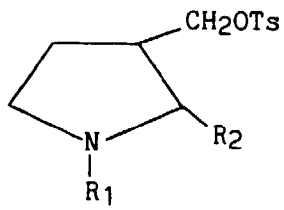

(VI)

dans laquelle $R_1$ et $R_2$ ont la même signification que dans la formule (I),

sur lequel on fait réagir du cyanure de potassium en milieu diméthylsulfoxyde,

pour conduire à la pyrrolidine de formule (VII) :

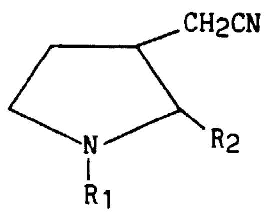

(VII)

dans laquelle $R_1$ et $R_2$ ont la même signification que dans la formule (I),

que l'on réduit à l'aide d'hydrure de diisobutylaluminium dans du toluène,

pour conduire à l'aldéhyde correspondant de formule (VIII) :

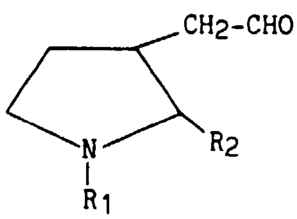

(VIII)

dans laquelle $R_1$ et $R_2$ ont la même signification que dans la formule (I),

b ou bien :

en présence d'un équivalent d'hydrure de diisobutylaluminium dans du toluène,

pour conduire à la pyrrolidine de formule (IX) :

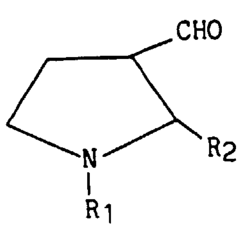

(IX)

dans laquelle $R_1$ et $R_2$ ont la même signification que dans la formule (I),

composés de formule (VIII) ou (IX) que l'on fait réagir avec un ylure de phosphore de formule (X), préparé par réaction du sel de phosphonium correspondant, en présence de terbutanolate de potassium dans du tétrahydrofurane,

$$(C_6H_5)_3\,P = CH\text{-}(CH_2)_m\text{-}\,CO_2H \qquad (X)$$

dans laquelle m a la même signification que dans la formule (I),

pour conduire respectivement aux composés de formule (I/a) ou (I/b), cas particulier des composés de formule (I) :

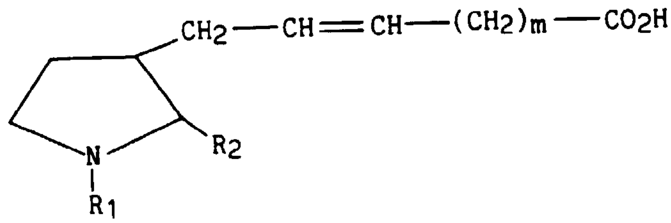

(I/a)

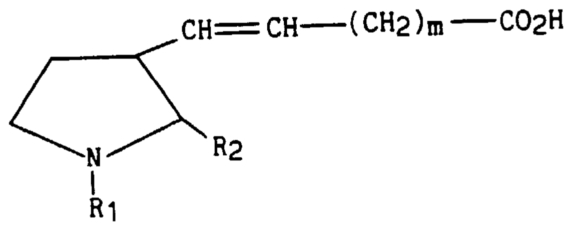

(I/b)

dans lesquelles $R_1$, $R_2$ et m ont la même signification que dans la formule (I),

composés de formule (I/a) ou (I/b)

- que l'on estérifie, si on le souhaite, pour conduire aux composés de formule ($I/a_1$) et ($I/b_1$) correspondants :

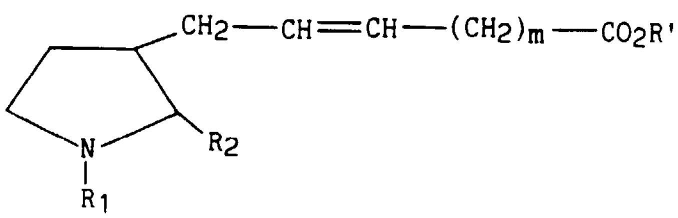

($I/a_1$)

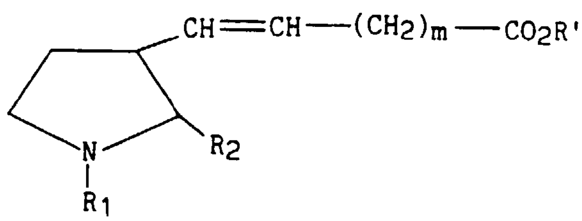

($I/b_1$)

dans lesquelles $R_1$ et $R_2$ ont la même signification que dans la formule (I) et R' représente un groupement alkyle ($C_1$-$C_6$) linéaire ou ramifié,

- dont on réduit éventuellement la double liaison par hydrogénation catalytique pour conduire au composé de formule (I/c), cas particulier des composés de formule (I),

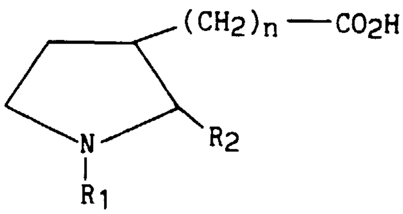

(I/c)

dans laquelle $R_1$, $R_2$ et n ont la même signification que dans la formule (I) et que l'on transforme, si on le souhaite, en ester correspondant de formule (I/$c_1$) :

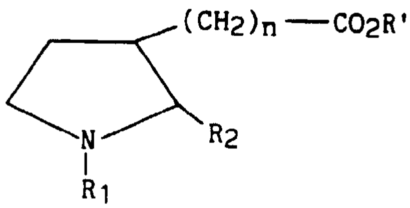

(I/c1)

dans laquelle $R_1$ et $R_2$ ont la même signification que dans la formule (I) et R' représente un groupement alkyle ($C_1$-$C_6$) linéaire ou ramifié, composés de formule (I/a), (I/$a_1$), (I/b), (I/$b_1$), (I/c), (I/$c_1$) qui constituent l'ensemble des composés de formule (I),

- qui, lorsque $R_2$ représente un noyau phényle substitué par un groupement alkoxy ($C_1$-$C_6$) linéaire ou ramifié, peuvent être transformés, si on le désire, en composés de formule (I) dans lesquels $R_2$ représente un noyau phényle substitué par un groupement hydroxy,
- que l'on purifie, le cas échéant, selon une technique classique de purification,
- dont on sépare éventuellement les isomères selon une technique classique de séparation,
- et que l'on transforme, si on le souhaite, en leurs sels d'addition à un acide ou à une base pharmaceutiquement acceptable.

**7.** Compositions pharmaceutiques contenant comme principe actif au moins un composé selon l'une quelconque des revendications 1 à 5, seul ou en combinaison avec un ou plusieurs véhicules inertes non toxiques pharmaceutiquement acceptables.

**8.** Composition pharmaceutique selon la revendication 7 contenant au moins un principe actif selon l'une des revendications 1 à 5 utiles en tant qu'antagonistes des récepteurs au thromboxane $A_2$ ou en tant qu'inhibiteurs de la thromboxane $A_2$-synthase.

**Revendications pour l'Etat contractant suivant : ES**

**1.** Procédé de préparation des composés de formulé (I) :

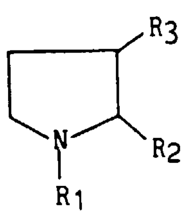

(I)

dans laquelle :
$R_1$ représente :

- un groupement alkyl ($C_1$-$C_6$) linéaire ou ramifié substitué ou non par un groupement pyridin-2-yl, pyridin-3-yl, phényl (lui-même éventuellement substitué par un ou plusieurs atomes d'halogène ou groupements alkyle

($C_1$-$C_6$) linéaire ou ramifié, alkoxy ($C_1$-$C_6$) linéaire ou ramifié, trihalogénométhyle),

- un groupement phényle substitué ou non par un ou plusieurs atomes d'halogène ou groupements alkyle ($C_1$-$C_6$) linéaire ou ramifié, alkoxy ($C_1$-$C_6$) linéaire ou ramifié, trihalogénométhyle,
- un groupement pyridyle,
- un groupement phénylsulfonyl substitué ou non sur le noyau phényle par un ou plusieurs atomes d'halogène ou groupements alkyle ($C_1$-$C_6$) linéaire ou ramifié, alkoxy ($C_1$-$C_6$) linéaire ou ramifié, trihalogénométhyle,
- un groupement acyle ($C_1$-$C_6$) linéaire ou ramifié,
- un groupement alkoxycarbonyl ($C_1$-$C_6$) linéaire ou ramifié,
- un groupement benzoyle (substitué ou non sur le noyau phényl par un ou plusieurs atomes d'halogène ou groupements hydroxy, alkyle, alkoxy, trifluorométhyle) ou pyridylcarbonyl,
- un groupement alkylaminocarbonyle ou phénylaminocarbonyle (substitué ou non sur le noyau phényl par un ou plusieurs atomes d'halogène ou groupements hydroxy, alkyle, alkoxy, trifluorométhyle),
- un groupement acylamino ou benzoylamino,

$R_2$ représente :

- un groupement phényle substitué ou non par un ou plusieurs atomes d'halogène ou groupements alkyle ($C_1$-$C_6$) linéaire ou ramifié, alkoxy ($C_1$-$C_6$) linéaire ou ramifié, hydroxy, trihalogénométhyle,
- un groupement pyridin-3-yle ou pyridin-2-yle,

$R_3$ représente l'un quelconque des groupements suivants :

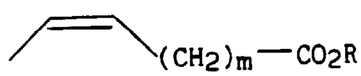

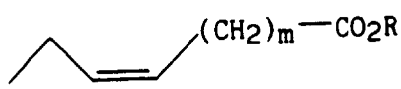

$-(CH_2)_n-CO_2R$

dans lesquels

m est égal à 2, 3 ou 4,

n est égal à 4, 5, 6 ou 7,

et R représente un atome d'hydrogène ou un groupement alkyle ($C_1$-$C_6$) linéaire ou ramifié,

leurs énantiomères, diastéréoisomères et épimères ainsi que leurs sels d'addition à un acide ou à une base pharmaceutiquement acceptable, caractérisé en ce que l'on utilise comme produit de départ une pyrrolidine de formule (II) :

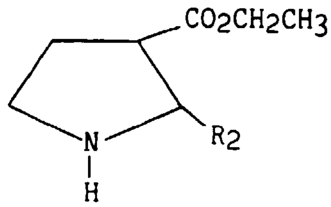

(II)

dans laquelle $R_2$ a la même signification que dans la formule (I), sous forme racémique ou dont on a préalablement séparé les isomères selon une technique classique de séparation,
que l'on fait réagir :

- soit avec de l'hydrure de lithium aluminium dans de l'éther, pour conduire à la pyrrolidine de formule (III) :

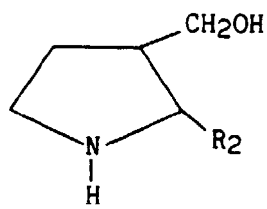

(III)

dans laquelle $R_2$ a la même signification que dans la formule (I),
que l'on met alors en réaction avec un dérivé halogéné de formule $R_1X$ dans laquelle $R_1$ a la même signification que dans la formule (I) et X représente un atome d'halogène, dans du chloroforme en présence de triéthylamine ou dans de l'acétonitrile en présence de carbonate de potassium,
pour conduire à la pyrrolidine de formule (IV) :

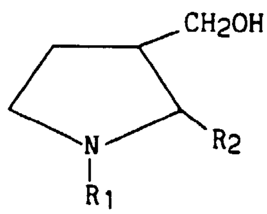

(IV)

dans laquelle $R_1$ et $R_2$ ont la même signification que dans la formule (I),

- soit avec un dérivé halogéné de formule $R_1X$ tel que défini précédemment,
pour conduire à la pyrrolidine de formule (V) :

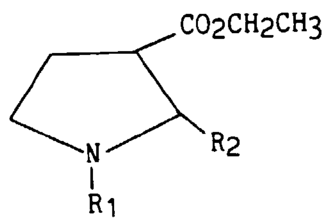

(V)

dans laquelle $R_1$ et $R_2$ ont la même signification que dans la formule (I),
composé de formule (V) qui, lorsqu'il est sous forme d'un couple d'énantiomères, peut être transformé, si on le souhaite, en son autre couple d'énantiomères par réaction de l'éthanolate de sodium, en milieu éthanolique, composé de formule (V) que l'on réduit :

a ou bien :

en présence de deux équivalents d'hydrure de diisobutylaluminium dans du toluène,

pour conduire à la pyrrolidine de formule (IV) définie précédemment, composé de formule (IV), qui, quelle que soit la voie de synthèse par lequel il a été obtenu, est transformé en tosylate (Ts) correspondant de formule (VI), par réaction avec du chlorure de toluène sulfonyle en présence de pyridine,

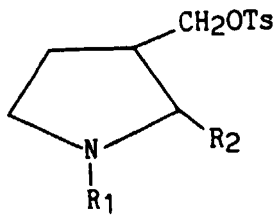

(VI)

dans laquelle $R_1$ et $R_2$ ont la même signification que dans la formule (I),

sur lequel on fait réagir du cyanure de potassium en milieu diméthylsulfoxyde,

pour conduire à la pyrrolidine de formule (VII) :

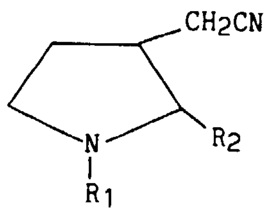

(VII)

dans laquelle $R_1$ et $R_2$ ont la même signification que dans la formule (I),

que l'on réduit à l'aide d'hydrure de diisobutylaluminium dans du toluène,

pour conduire à l'aldéhyde correspondant de formule (VIII) :

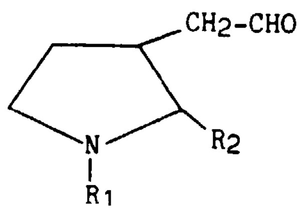

(VIII)

dans laquelle $R_1$ et $R_2$ ont la même signification que dans la formule (I),

b ou bien :

en présence d'un équivalent d'hydrure de diisobutylaluminium dans du toluène,

pour conduire à la pyrrolidine de formule (IX) :

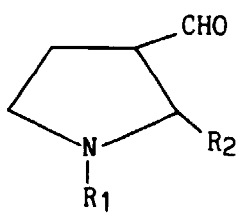

(IX)

dans laquelle $R_1$ et $R_2$ ont la même signification que dans la formule (I),

composés de formule (VIII) ou (IX) que l'on fait réagir avec un ylure de phosphore de formule (X), préparé par réaction du sel de phosphonium correspondant, en présence de terbutanolate de potassium dans du tétrahydrofurane,

$$(C_6H_5)_3\ P{=}CH\text{-}(CH_2)_m\text{-}CO_2H \qquad (X)$$

dans laquelle m a la même signification que dans la formule (I),

pour conduire respectivement aux composés de formule (I/a) ou (I/b), cas particulier des composés de formule (I) :

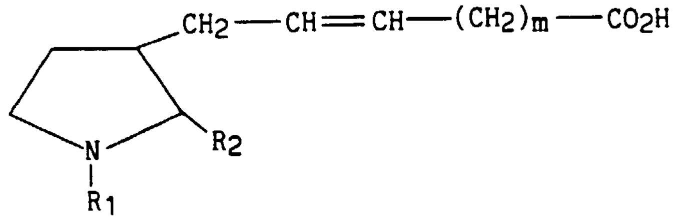

(I/a)

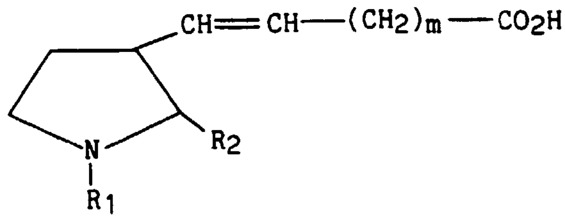

(I/b)

dans lesquelles $R_1$, $R_2$ et m ont la même signification que dans la formule (I),

composés de formule (I/a) ou (I/b)

- que l'on estérifie, si on le souhaite, pour conduire aux composés de formule ($I/a_1$) et ($I/b_1$) correspondants :

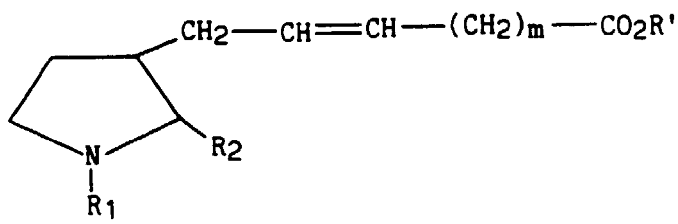

($I/a_1$)

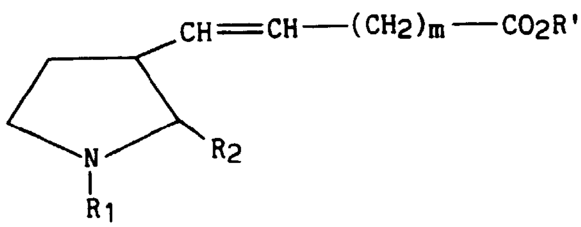

($I/b_1$)

dans lesquelles $R_1$ et $R_2$ ont la même signification que dans la formule (I) et R' représente un groupement alkyle ($C_1$-$C_6$) linéaire ou ramifié,

- dont on réduit éventuellement la double liaison par hydrogénation catalytique pour conduire au composé de formule (I/c), cas particulier des composés de formule (I),

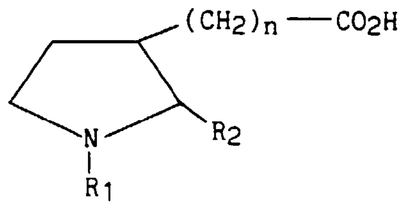

(I/c)

dans laquelle $R_1$, $R_2$ et n ont la même signification que dans la formule (I) et que l'on transforme, si on le souhaite, en ester correspondant de formule ($I/c_1$) :

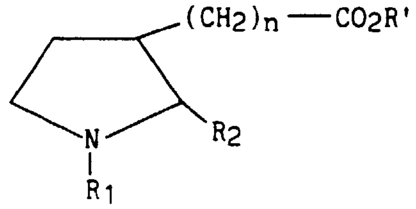

($I/c_1$)

dans laquelle $R_1$ et $R_2$ ont la même signification que dans la formule (I) et R' représente un groupement alkyle ($C_1$-$C_6$) linéaire ou ramifié,

composés de formule (I/a), ($I/a_1$), (I/b), ($I/b_1$), (I/c), ($I/c_1$) qui constituent l'ensemble des composés de formule (I),

- qui, lorsque $R_2$ représente un noyau phényle substitué par un groupement alkoxy ($C_1$-$C_6$) linéaire ou ramifié, peuvent être transformés, si on le désire, en composés de formule (I) dans lesquels $R_2$ représente un noyau phényle substitué par un groupement hydroxy,
- que l'on purifie, le cas échéant, selon une technique classique de purification,
- dont on sépare éventuellement les isomères selon une technique classique de séparation,
- et que l'on transforme, si on le souhaite, en leurs sels d'addition à un acide ou à une base pharmaceutiquement acceptable.

**2.** Procédé de préparation des composés de formule (I) selon la revendication 1 dans laquelle $R_1$ représente un groupement phénylsulfonyl substitué ou non, leurs énantiomères, diastéréoisomères et épimères ainsi que leurs sels d'addition à un acide ou à une base pharmaceutiquement acceptable.

**3.** Procédé de préparation des composés de formule (I) selon la revendication 1 dans laquelle $R_1$ représente un groupement pyridin-3-yl-méthyl, leurs énantiomères, diastéréoisomères et épimères ainsi que leurs sels d'addition à un acide ou à une base pharmaceutiquement acceptable.

**4.** Procédé de préparation des composés de formule (I) selon la revendication 1 dans laquelle $R_2$ représente un groupement phényle substitué ou non, leurs énantiomères, diastéréoisomères et épimères ainsi que leurs sels d'addition à un acide ou à une base pharmaceutiquement acceptable.

**5.** Procédé de préparation du composé de formule (I) selon l'une quelconque des revendications 1, 3 ou 4 qui est l'acide (4Z)-6-[(2α,3α)-1-[(pyridin-3-yl)méthyl]-2-phénylpyrrolidin-3-yl]hex-4-ènoïque, ses énantiomères, diastéréoisomères ainsi que ses sels d'addition à une base ou un acide pharmaceutiquement acceptable.

## Patentansprüche

### Patentansprüche für folgende Vertragsstaaten: AT, BE, CH, DE, DK, FR, GB, GR, IE, IT, LI, LU, NL, PT, SE

**1.** Verbindungen der Formel (I):

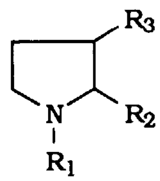

(I)

in der:

$R_1$ :

- eine geradkettige oder verzweigte ($C_1$-$C_6$)-Alkylgruppe, die gegebenenfalls durch eine Pyridin-2-yl-gruppe, Pyridin-3-yl-gruppe oder Phenylgruppe (die ihrerseits gegebenenfalls durch eines oder mehrere Halogenatome oder geradkettige oder verzweigte ($C_1$-$C_6$)-Alkylgruppen, geradkettige oder verzweigte ($C_1$-$C_6$)-Alkoxygruppen oder Trihalogenomethylgruppen substituiert ist) substituiert ist,
- eine Phenylgruppe, die gegebenenfalls durch eines oder mehrere Halogenatome oder geradkettige oder verzweigte ($C_1$-$C_6$)-Alkylgruppen, geradkettige oder verzweigte ($C_1$-$C_6$)-Alkoxygruppen oder Trihalogenomethylgruppen substituiert ist,
- eine Pyridylgruppe,
- eine Phenylsulfonylgruppe, die gegebenenfalls am Phenylkern durch eines oder mehrere Halogenatome oder geradkettige oder verzweigte ($C_1$-$C_6$)-Alkylgruppen, geradkettige oder verzweigte ($C_1$-$C_6$)-Alkoxygruppen oder Trihalogenomethylgruppen substituiert ist,
- eine geradkettige oder verzweigte ($C_1$-$C_6$)-Acylgruppe,
- eine geradkettige oder verzweigte ($C_1$-$C_6$)-Alkoxycarbonylgruppe,
- eine Benzoylgruppe (die gegebenenfalls am Phenylkern durch eines oder mehrere Halogenatome oder Hydroxylgruppen, Alkylgruppen, Alkoxygruppen oder Trifluormethylgruppen substituiert ist) oder eine Pyridylcarbonylgruppe,
- eine Alkylaminocarbonylgruppe oder Phenylaminocarbonylgruppe (die gegebenenfalls am Phenylkern durch eines oder mehrere Halogenatome oder Hydroxyl-, Alkyl-, Alkoxy- oder Trifluormethylgruppen substituiert ist),
- eine Acylamino- oder Benzoylaminogruppe,

$R_2$:

- eine Phenylgruppe, die gegebenenfalls durch eines oder mehrere Halogenatome oder geradkettige oder verzweigte ($C_1$-$C_6$)-Alkylgruppen, geradkettige oder verzweigte ($C_1$-$C_6$)-Alkoxygruppen, Hydroxylgruppen oder Trihalogenomethylgruppen substituiert ist,
- eine Pyridin-3-yl- oder Pyridin-2-yl-gruppe,

$R_3$ eine der folgenden Gruppen:

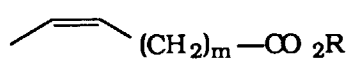

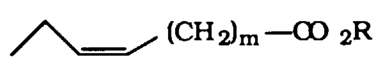

$-(CH_2)_n-CO_2R$

in denen

m 2, 3 oder 4,
n 4, 5, 6 oder 7 und
R ein Wasserstoffatom oder eine geradkettige oder verzweigte ($C_1$-$C_6$)-Alkylgruppe darstellen,

bedeuten, deren Enantiomere, Diastereoisomere und Epimere sowie deren Additionssalze mit einer pharmazeutisch annehmbaren Säure oder Base.

**2.** Verbindungen der Formel (I) nach Anspruch 1, in der $R_1$ eine gegebenenfalls substituierte Phenylsulfonylgruppe

darstellt, deren Enantiomere, Diastereoisomere und Epimere sowie deren Additionssalze mit einer pharmazeutisch annehmbaren Säure oder Base.

**3.** Verbindungen der Formel (I) nach Anspruch 1, in der $R_1$ eine Pyridin-3-yl-methylgruppe bedeutet, deren Enantiomere, Diastereoisomere und Epimere sowie deren Additionssalze mit einer pharmazeutisch annehmbaren Säure oder Base.

**4.** Verbindungen der Formel (I) nach Anspruch 1, in der $R_2$ eine gegebenenfalls substituierte Phenylgruppe bedeutet, deren Enantiomere, Diastereoisomere und Epimere sowie derenAdditionssalze mit einer pharmazeutisch annehmbaren Säure oder Base.

**5.** Verbindung der Formel (I) nach einem der Ansprüche 1, 3 oder 4, nämlich (4Z)-6-[(2α,3α)-1[(pyridin-3-yl)-methyl]-2-phenylpyrrolidin-3-yl]-hex-4-ensäure, deren Enantiomere, Diastereoisomere sowie deren Additionssalze mit einer pharmazeutisch annehmbaren Base oder Säure.

**6.** Verfahren zur Herstellung der Derivate der Formel (I) nach Anspruch 1, **dadurch gekennzeichnet,** daß man als Ausgangsprodukt ein Pyrrolidin der Formel (II):

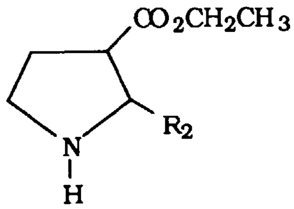

(II)

in der $R_2$ die bezüglich der Formel (I) angegebenen Bedeutungen besitzt, in racemischer Form oder nach der Trennung in seine Isomeren mit Hilfe einer klassischen Trennmethode verwendet,
welches man:

- entweder mit Lithiumaluminiumhydrid in Ether umsetzt zur Bildung des Pyrrolidins der Formel (III):

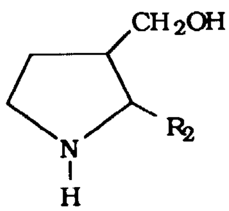

(III)

in der $R_2$ die bezüglich der Formel (I) angegebenen Bedeutungen besitzt, welches man mit einem Halogenderivat der Formel $R_1X$, in der $R_1$ die bezüglich der Formel (I) angegebenen Bedeutungen besitzt und X ein Halogenatom darstellt, in Chloroform in Gegenwart von Triethylamin oder in Acetonitril in Gegenwart von Kaliumcarbonat umsetzt,
so daß man das Pyrrolidin der Formel (IV) erhält:

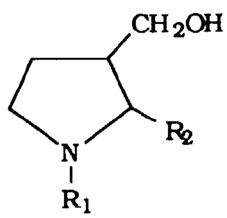

(IV)

in der $R_1$ und $R_2$ die bezüglich der Formel (I) angegebenen Bedeutungen besitzen,

- oder mit einem Halogenderivat der Formel $R_1X$, wie es oben definiert worden ist,
zur Bildung des Pyrrolidins der Formel (V):

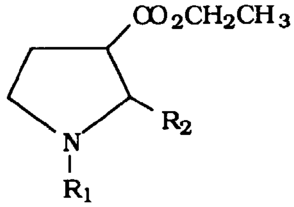

(V)

in der $R_1$ und $R_2$ die bezüglich der Formel (I) angegebenen Bedeutungen besitzen,
welche Verbindung der Formel (V), wenn sie in Form eines Enantiomerenpaars vorliegt, gewünschtenfalls durch Reaktion mit Natriumethanolat in ethanolischem Medium in das andere Enantiomerenpaar umgewandelt werden kann,
welche Verbindung der Formel (V) man reduziert:

a entweder:
in Gegenwart von zwei Äquivalenten Diisobutylaluminiumhydrid in Toluol, so daß man das oben definierte Pyrrolidin der Formel (IV) erhält, welche Verbindung der Formel (IV), unabhängig von dem Syntheseweg, auf dem sie hergestellt worden ist, durch Reaktion mit Toluolsulfonylchlorid in Gegenwart von Pyridin in das entsprechende Tosylat (Ts) der Formel (VI) umgewandelt wird:

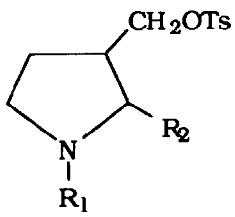

(VI)

in der $R_1$ und $R_2$ die bezüglich der Formel (I) angegebenen Bedeutungen besitzen,
welches man mit Kaliumcyanid in Dimethylsulfoxidmedium umsetzt, zur Bildung des Pyrrolidins der Formel (VII):

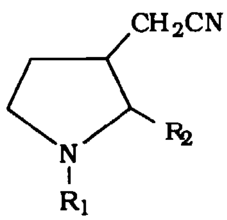

(VII)

in der $R_1$ und $R_2$ die bezüglich der Formel (I) angegebenen Bedeutungen besitzen,
welches man mit Diisobutylaluminiumhydrid in Toluol reduziert zur Bildung des entsprechenden Aldehyds der Formel (VIII):

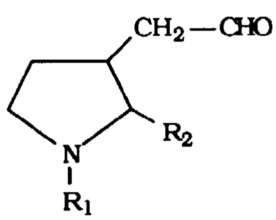

(VIII)

in der $R_1$ und $R_2$ die bezüglich der Formel (I) angegebenen Bedeutungen besitzen.
b oder:
in Gegenwart eines Äquivalents Diisobutylaluminiumhydrid in Toluol, zur Bildung des Pyrrolidins der Formel (IX):

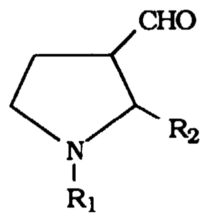

(IX)

in der $R_1$ und $R_2$ die bezüglich der Formel (I) angegebenen Bedeutungen besitzen,

welche Verbindungen der Formeln (VIII) oder (IX) man mit einem durch Reaktion des entsprechenden Phosphoniumsalzes gebildeten Phosphorylid der Formel (X)

$$(C_6H_5)_3\ P = CH - (CH_2)_m - CO_2H \qquad (X)$$

in der m die bezüglich der Formel (I) angegebenen Bedeutungen besitzt, in Gegenwart von Kalium-tert.-butanolat in Tetrahydrofuran umsetzt,

zur Bildung der Verbindungen der Formel (I/a) bzw. (I/b), einem Sonderfall der Verbindungen der Formel (I):

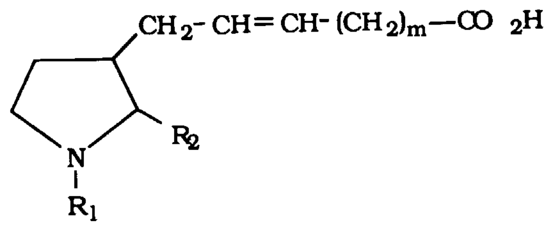

(I/a)

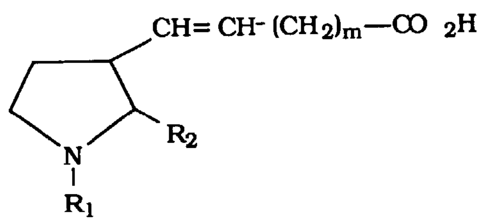

(I/b)

in denen $R_1$, $R_2$ und m die bezüglich der Formel (I) angegebenen Bedeutungen besitzen,

welche Verbindungen der Formel (I/a) oder (I/b)

- man gewünschtenfalls verestert zur Bildung der entsprechenden Verbindungen der Formel ($I/a_1$) und ($I/b_1$):

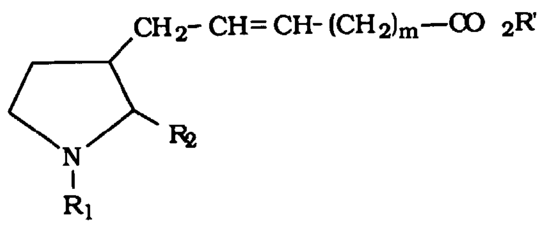

($I/a_1$)

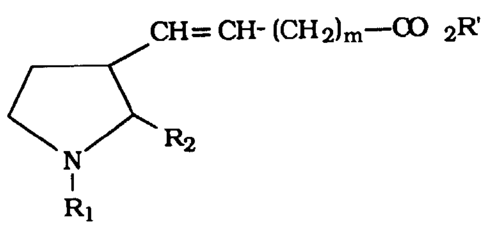

($I/b_1$)

in denen $R_1$ und $R_2$ die bezüglich der Formel (I) angegebenen Bedeutungen besitzen und R' eine geradkettige oder verzweigte ($C_1$-$C_6$)-Alkylgruppe darstellt,

- deren Doppelbindung man gegebenenfalls durch katalytische Hydrierung reduziert zur Bildung der Verbindung der Formel (I/c), einem Sonderfall der Verbindungen der Formel (I):

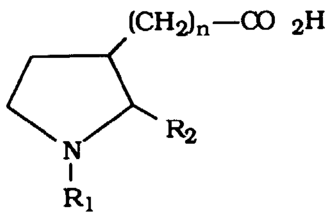

(I/c)

in der $R_1$, $R_2$ und n die bezüglich der Formel (I) angegebenen Bedeutungen besitzen, welche man gewünschtenfalls in den entsprechenden Ester der Formel ($I/c_1$) umwandelt:

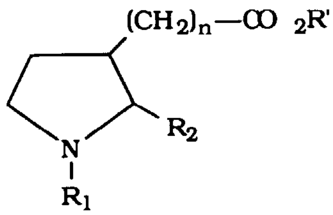

($I/c_1$)

in der $R_1$ und $R_2$ die bezüglich der Formel (I) angegebenen Bedeutungen besitzen und R' eine geradkettige oder verzweigte ($C_1$-$C_6$)-Alkylgruppe darstellt,

welche Verbindungen der Formeln (I/a), ($I/a_1$), (I/b), ($I/b_1$), (I/c) und ($I/c_1$) gemeinsam die Verbindungen der Formel (I) bilden,

- welche, wenn $R_2$ einen durch eine geradkettige oder verzweigte ($C_1$-$C_6$)-Alkoxygruppe substituierten Phenylkern darstellt, gewünschtenfalls in die Verbindungen der Formel (I), in der $R_2$ einen durch eine Hydroxylgruppe substituierten Phenylkern bedeutet, umgewandelt werden können,
- welche man gegebenenfalls mit Hilfe einer klassischen Reinigungstechnik reinigt,
- welche man gegebenenfalls mit Hilfe einer klassischen Trennungsmethode in ihre Isomeren auftrennt und
- welche man gewünschtenfalls mit einer pharmazeutisch annehmbaren Säure oder Base in ihre Additionssalze umwandelt.

7. Pharmazeutische Zubereitungen, enthaltend als Wirkstoff mindestens eine Verbindung nach einem der Ansprüche 1 bis 5 allein oder in Kombination mit einem oder mehreren inerten, nichttoxischen, pharmazeutisch annehmbaren Trägermaterialien.

8. Pharmazeutische Zubereitungen nach Anspruch 7, enthaltend mindestens einen Wirkstoff nach einem der Ansprüche 1 bis 5 als Antagonisten von Thromboxan $A_2$-Rezeptoren oder als Inhibitoren der Thromboxan $A_2$-Synthase.

**Patentansprüche für folgenden Vertragsstaat : ES**

1. Verfahren zur Herstellung der Verbindungen der Formel (I):

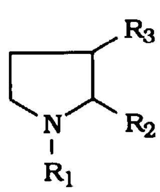

(I)

in der:
$R_1$:

- eine geradkettige oder verzweigte ($C_1$-$C_6$)-Alkylgruppe, die gegebenenfalls durch eine Pyridin-2-yl-gruppe, Pyridin-3-yl-gruppe oder Phenylgruppe (die ihrerseits gegebenenfalls durch eines oder mehrere Halogenatome oder geradkettige oder verzweigte ($C_1$-$C_6$)-Alkylgruppen, geradkettige oder verzweigte ($C_1$-$C_6$)-Alkoxygruppen oder Trihalogenomethylgruppen substituiert ist) substituiert ist,

- eine Phenylgruppe, die gegebenenfalls durch eines oder mehrere Halogenatome oder geradkettige oder verzweigte ($C_1$-$C_6$)-Alkylgruppen, geradkettige oder verzweigte ($C_1$-$C_6$)-Alkoxygruppen oder Trihalogenomethylgruppen substituiert ist,
- eine Pyridylgruppe,
- eine Phenylsulfonylgruppe, die gegebenenfalls am Phenylkern durch eines oder mehrere Halogenatome oder geradkettige oder verzweigte ($C_1$-$C_6$)-Alkylgruppen, geradkettige oder verzweigte ($C_1$-$C_6$)-Alkoxygruppen oder Trihalogenomethylgruppen substituiert ist,
- eine geradkettige oder verzweigte ($C_1$-$C_6$)-Acylgruppe,
- eine geradkettige oder verzweigte ($C_1$-$C_6$)-Alkoxycarbonylgruppe,
- eine Benzoylgruppe (die gegebenenfalls am Phenylkern durch eines oder mehrere Halogenatome oder Hydroxylgruppen, Alkylgruppen, Alkoxygruppen oder Trifluormethylgruppen substituiert ist) oder eine Pyridylcarbonylgruppe,
- eine Alkylaminocarbonylgruppe oder Phenylaminocarbonylgruppe (die gegebenenfalls am Phenylkern durch eines oder mehrere Halogenatome oder Hydroxyl-, Alkyl-, Alkoxy- oder Trifluormethylgruppen substituiert ist),
- eine Acylamino- oder Benzoylaminogruppe,

$R_2$:

- eine Phenylgruppe, die gegebenenfalls durch eines oder mehrere Halogenatome oder geradkettige oder verzweigte ($C_1$-$C_6$)-Alkylgruppen, geradkettige oder verzweigte ($C_1$-$C_6$)-Alkoxygruppen, Hydroxylgruppen oder Trihalogenomethylgruppen substituiert ist,
- eine Pyridin-3-yl- oder Pyridin-2-yl-gruppe,

$R_3$ eine der folgenden Gruppen:

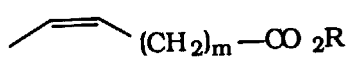

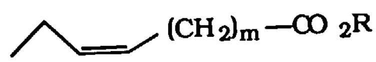

$-(CH_2)_n-CO_2R$

in denen

m 2, 3 oder 4,
n 4, 5, 6 oder 7 und
R ein Wasserstoffatom oder eine geradkettige oder verzweigte ($C_1$-$C_6$)-Alkylgruppe darstellen,

bedeuten, von deren Enantiomeren, Diastereoisomeren und Epimeren sowie von deren Additionssalzen mit einer pharmazeutisch annehmbaren Säure oder Base, **dadurch gekennzeichnet,** daß man als Ausgangsprodukt ein Pyrrolidin der Formel (II):

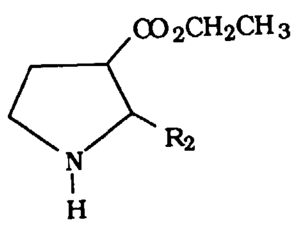

(II)

in der $R_2$ die bezüglich der Formel (I) angegebenen Bedeutungen besitzt, in racemischer Form oder nach der Trennung in seine Isomeren mit Hilfe einer klassischen Trennmethode verwendet,
welches man:

- entweder mit Lithiumaluminiumhydrid in Ether umsetzt zur Bildung des Pyrrolidins der Formel (III):

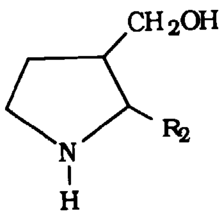

(III)

in der $R_2$ die bezüglich der Formel (I) angegebenen Bedeutungen besitzt, welches man mit einem Halogenderivat der Formel $R_1X$, in der $R_1$ die bezüglich der Formel (I) angegebenen Bedeutungen besitzt und X ein Halogenatom darstellt, in Chloroform in Gegenwart von Triethylamin oder in Acetonitril in Gegenwart von Kaliumcarbonat umsetzt,
so daß man das Pyrrolidin der Formel (IV) erhält:

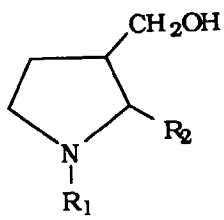

(IV)

in der $R_1$ und $R_2$ die bezüglich der Formel (I) angegebenen Bedeutungen besitzen,

- oder mit einem Halogenderivat der Formel $R_1X$, wie es oben definiert worden ist,
zur Bildung des Pyrrolidins der Formel (V):

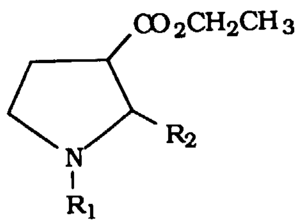

(V)

in der $R_1$ und $R_2$ die bezüglich der Formel (I) angegebenen Bedeutungen besitzen,
welche Verbindung der Formel (V), wenn sie in Form eines Enantiomerenpaars vorliegt, gewünschtenfalls durch Reaktion mit Natriumethanolat in ethanolischem Medium in das andere Enantiomerenpaar umgewandelt werden kann,
welche Verbindung der Formel (V) man reduziert:

a entweder:
in Gegenwart von zwei Äquivalenten Diisobutylaluminiumhydrid in Toluol, so daß man das oben definierte Pyrrolidin der Formel (IV) erhält, welche Verbindung der Formel (IV), unabhängig von dem Syntheseweg, auf dem sie hergestellt worden ist, durch Reaktion mit Toluolsulfonylchlorid in Gegenwart von Pyridin in das entsprechende Tosylat (Ts) der Formel (VI) umgewandelt wird:

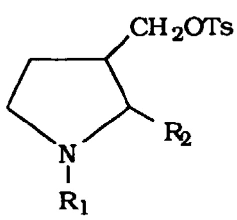

(VI)

in der $R_1$ und $R_2$ die bezüglich der Formel (I) angegebenen Bedeutungen besitzen,
welches man mit Kaliumcyanid in Dimethylsulfoxidmedium umsetzt, zur Bildung des Pyrrolidins der Formel (VII):

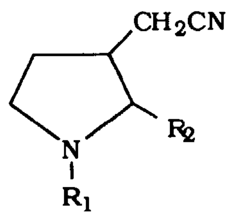

(VII)

in der $R_1$ und $R_2$ die bezüglich der Formel (I) angegebenen Bedeutungen besitzen, welches man mit Diisobutylaluminiumhydrid in Toluol reduziert zur Bildung des entsprechenden Aldehyds der Formel (VIII):

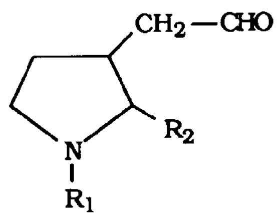

(VIII)

in der $R_1$ und $R_2$ die bezüglich der Formel (I) angegebenen Bedeutungen besitzen,
b oder:
in Gegenwart eines Äquivalents Diisobutylaluminiumhydrid in Toluol, zur Bildung des Pyrrolidins der Formel (IX):

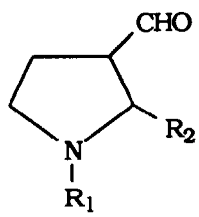

(IX)

in der $R_1$ und $R_2$ die bezüglich der Formel (I) angegebenen Bedeutungen besitzen, welche Verbindungen der Formeln (VIII) oder (IX) man mit einem durch Reaktion des entsprechenden Phosphoniumsalzes gebildeten Phosphorylid der Formel (X)

$$(C_6H_5)_3 P = CH - (CH_2)_m - CO_2H \quad (X)$$

in der m die bezüglich der Formel (I) angegebenen Bedeutungen besitzt, in Gegenwart von Kalium-tert.-butanolat in Tetrahydrofuran umsetzt,
zur Bildung der Verbindungen der Formel (I/a) bzw. (I/b), einem Sonderfall der Verbindungen der Formel (I):

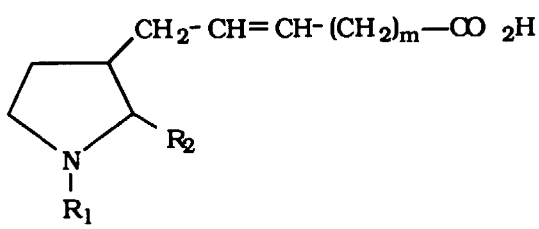

(I/a)

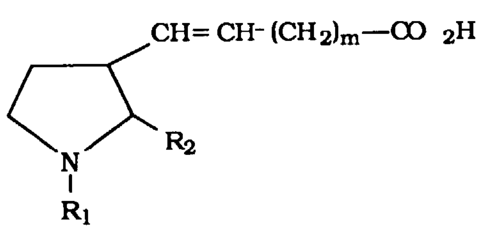

(I/b)

in denen $R_1$, $R_2$ und m die bezüglich der Formel (I) angegebenen Bedeutungen besitzen, welche Verbindungen der Formel (I/a) oder (I/b)

- man gewünschtenfalls verestert zur Bildung der entsprechenden Verbindungen der Formel (I/$a_1$) und (I/$b_1$):

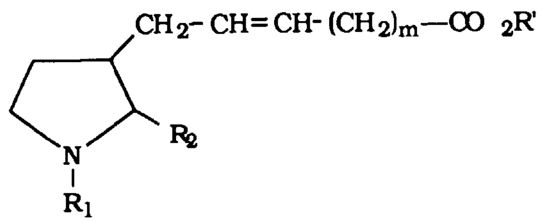

(I/$a_1$)

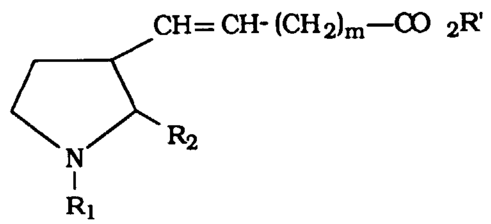

(I/$b_1$)

in denen $R_1$ und $R_2$ die bezüglich der Formel (I) angegebenen Bedeutungen besitzen und R' eine geradkettige oder verzweigte ($C_1$-$C_6$)-Alkylgruppe darstellt,

- deren Doppelbindung man gegebenenfalls durch katalytische Hydrierung reduziert zur Bildung der Verbindung der Formel (I/c), einem Sonderfall der Verbindungen der Formel (I):

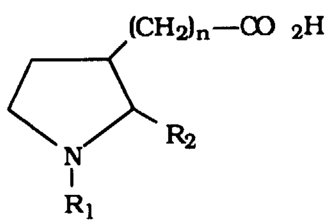

(I/c)

in der $R_1$, $R_2$ und n die bezüglich der Formel (I) angegebenen Bedeutungen besitzen, welche man gewünschtenfalls in den entsprechenden Ester der Formel (I/$c_1$) umwandelt:

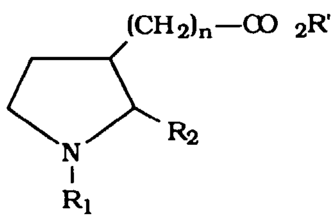

(I/$c_1$)

in der $R_1$ und $R_2$ die bezüglich der Formel (I) angegebenen Bedeutungen besitzen und R' eine geradkettige oder verzweigte ($C_1$-$C_6$)-Alkylgruppe darstellt,

welche Verbindungen der Formeln (I/a), (I/$a_1$), (I/b), (I/$b_1$), (I/c) und (I/$c_1$) gemeinsam die Verbindungen der Formel (I) bilden.

- welche, wenn $R_2$ einen durch eine geradkettige oder verzweigte ($C_1$-$C_6$)-Alkoxygruppe substituierten Phenylkern darstellt, gewünschtenfalls in die Verbindungen der Formel (I), in der $R_2$ einen durch eine Hydroxylgruppe substituierten Phenylkern bedeutet, umgewandelt werden können,
- welche man gegebenenfalls mit Hilfe einer klassischen Reinigungstechnik reinigt,
- welche man gegebenenfalls mit Hilfe einer klassischen Trennungsmethode in ihre Isomeren auftrennt und
- welche man gewünschtenfalls mit einer pharmazeutisch annehmbaren Säure oder Base in ihre Additionssalze umwandelt.

**2.** Verfahren zur Herstellung der Verbindungen der Formel (I) nach Anspruch 1, worin $R_1$ eine gegebenenfalls substituierte Phenylgruppe bedeutete, von deren Enantiomeren, Diastereoisomeren und Epimeren sowie von deren Additionssalzen mit einer pharmazeutisch annehmbaren Säure oder Base.

**3.** Verfahren zur Herstellung der Verbindungen der Formel (I) nach Anspruch 1, worin $R_1$ eine Pyridin-3-yl-methylgruppe bedeutet, von deren Enantiomeren, Diastereoisomeren und Epimeren sowie von deren Additionssalzen mit einer pharmazeutisch annehmbaren Säure oder Base.

**4.** Verfahren zur Herstellung der Verbindungen der Formel (I) nach Anspruch 1, worin $R_2$ eine gegebenenfalls substituierte Phenylgruppe bedeutet, von deren Enantiomeren, Diastereoisomeren und Epimeren sowie von deren Additionssalzen mit einer pharmazeutisch annehmbaren Säure oder Base.

**5.** Verfahren zur Herstellung der Verbindung der Formel (I) nach einem der Ansprüche 1, 3 oder 4, nämlich von (4Z)-6-[(2α,3α)-1-[(pyridin-3-yl)-methyl]-2-phenylpyrrolidin-3-yl]-hex-4-ensäure, von deren Enantiomeren und Diastereoisomeren sowie von deren Additionssalzen mit einer pharmazeutisch annehmbaren Base oder Säure.

**Claims**

**Claims for the following Contracting States : AT, BE, CH, DE, DK, FR, GB, GR, IE, IT, LI, LU, NL, PT, SE**

**1.** Compounds of formula (I):

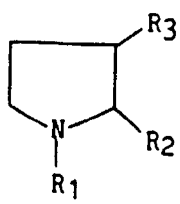

(I)

wherein :
$R_1$ represents :

- a straight-chained or branched ($C_1$-$C_6$)-alkyl group that is unsubstituted or substituted by a pyridin-2-yl, pyridin-3-yl or phenyl group (which is itself optionally substituted by one or more halogen atoms or groups straight-chained or branched ($C_1$-$C_6$)-alkyl, straight-chained or branched ($C_1$-$C_6$)-alkoxy, trihalomethyl),
- a phenyl group that is unsubstituted or substituted by one or more halogen atoms or groups straight-chained or branched ($C_1$-$C_6$)-alkyl, straight-chained or branched ($C_1$-$C_6$)-alkoxy, trihalomethyl,
- a pyridyl group,
- a phenylsulphonyl group that is unsubstituted or substituted at the phenyl nucleus by one or more halogen atoms or groups straight-chained or branched ($C_1$-$C_6$)-alkyl, straight-chained or branched ($C_1$-$C_6$)-alkoxy, trihalomethyl,
- a straight-chained or branched ($C_1$-$C_6$)-acyl group,
- a straight-chained or branched ($C_1$-$C_6$)-alkoxycarbonyl group,
- a benzoyl group (unsubstituted or substituted at the phenyl nucleus by one or more halogen atoms or groups hydroxy, alkyl, alkoxy, trifluoromethyl) or a pyridylcarbonyl group,
- an alkylaminocarbonyl or phenylaminocarbonyl group (unsubstituted or substituted at the phenyl nucleus by one or more halogen atoms or groups hydroxy, alkyl, alkoxy, trifluoromethyl),
- an acylamino or benzoylamino group,

$R_2$ represents :

- a phenyl group that is unsubstituted or substituted by one or more halogen atoms or groups straight-chained or branched ($C_1$-$C_6$)-alkyl, straight-chained or branched ($C_1$-$C_6$)-alkoxy, hydroxy, trihalomethyl,

- a pyridin-3-yl or pyridin-2-yl group,

$R_3$ represents any one of the following groupings :

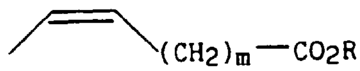

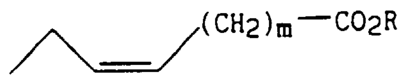

$-(CH_2)_n - CO_2R$

wherein

m is 2, 3 or 4,

n is 4, 5, 6 or 7,

and R represents a hydrogen atom or a straight-chained or branched $(C_1-C_6)$-alkyl group,

their enantiomers, diastereoisomers and epimers and also their addition salts with a pharmaceutically acceptable acid or base.

**2.** Compounds of formula (I) according to claim 1, wherein $R_1$ represents a substituted or unsubstituted phenylsulphonyl group, their enantiomers, diastereoisomers and epimers and also their addition salts with a pharmaceutically acceptable acid or base.

**3.** Compounds of formula (I) according to claim 1, wherein $R_1$ represents a pyridin-3-ylmethyl group, their enantiomers, diastereoisomers and epimers and also their addition salts with a pharmaceutically acceptable acid or base.

**4.** Compounds of formula (I) according to claim 1, wherein $R_2$ represents a substituted or unsubstituted phenyl group, their enantiomers, diastereoisomers and epimers and also their addition salts with a pharmaceutically acceptable acid or base.

**5.** Compound of formula (I) according to any one of claims 1, 3 and 4 which is (4Z)-6-[(2α,3α)-1-[(pyridin-3-yl)methyl]-2-phenylpyrrolidin-3-yl]hex-4-enoic acid, its enantiomers, diastereoisomers and also its addition salts with a pharmaceutically acceptable acid or base.

**6.** Process for the preparation of compounds of formula (I) according to claim 1, characterised in that there is used as starting material a pyrrolidine of formula (II) :

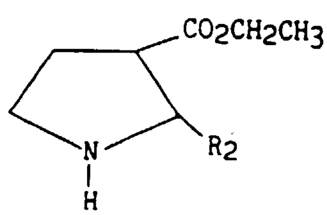

(II)

wherein $R_2$ is as defined for formula (I), in racemic form or which has previously been separated into its isomers according to a conventional method of separation,
which is reacted :

- either with lithium aluminium hydride in ether, to yield the pyrrolidine of formula (III) :

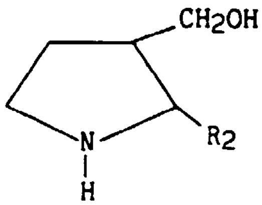

(III)

wherein $R_2$ is as defined for formula (I), which is then reacted with a halogenated compound of formula $R_1X$, wherein $R_1$ is as defined for formula (I) and X represents a halogen atom, in chloroform in the presence of triethylamine or in acetonitrile in the presence of potassium carbonate,
to yield the pyrrolidine of formula (IV) :

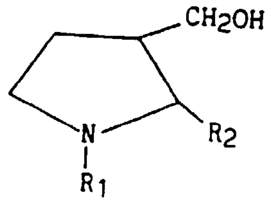

(IV)

wherein $R_1$ and $R_2$ are as defined for formula (I),

- or with a halogenated compound of formula $R_1X$ as defined above, to yield the pyrrolidine of formula (V) :

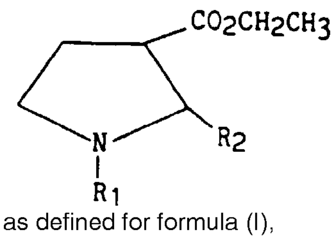

(V)

wherein $R_1$ and $R_2$ are as defined for formula (I),
which compound of formula (V), when it is in the form of a pair of enantiomers, may be converted, if desired, into its other pair of enantiomers by sodium ethanolate reaction in ethanolic medium,
which compound of formula (V) is reduced :

either a:

in the presence of two equivalents of diisobutylaluminium hydride in toluene,
to yield the pyrrolidine of formula (IV) defined above, which compound of formula (IV), whatever the method of synthesis by which it has been obtained, is converted by reaction with toluenesulphonyl chloride, in the presence of pyridine, into the corresponding tosylate (Ts) of formula (VI)

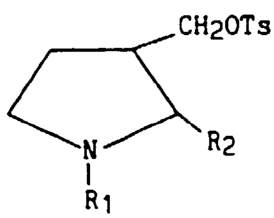

(VI)

wherein $R_1$ and $R_2$ are as defined for formula (I),
which is reacted with potassium cyanide in dimethyl sulphoxide medium,
to yield the pyrrolidine of formula (VII) :

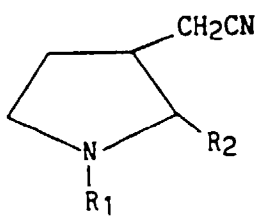

(VII)

wherein $R_1$ and $R_2$ are as defined for formula (I),
which is reduced with diisobutylaluminium hydride in toluene,
to yield the corresponding aldehyde of formula (VIII) :

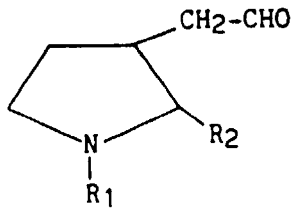

(VIII)

wherein $R_1$ and $R_2$ are as defined for formula (I),

or b:

in the presence of one equivalent of diisobutylaluminium hydride in toluene,

to yield the pyrrolidine of formula (IX) :

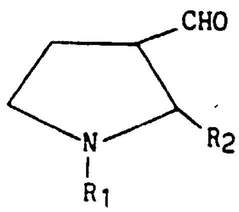

(IX)

wherein $R_1$ and $R_2$ are as defined for formula (I),

which compounds of formula (VIII) or (IX) are reacted with a phosphorus ylid of formula (X), prepared by reaction of the corresponding phosphonium salt, in the presence of potassium tert-butanolate in tetrahydrofuran,

$$(C_6H_5)_3\,P = CH\text{-}(CH_2)_m\text{-}CO_2H \qquad (X)$$

wherein m is as defined for formula (I),
to yield, respectively, the compounds of formula (I/a) or (I/b), a particular case of compounds of formula (I) :

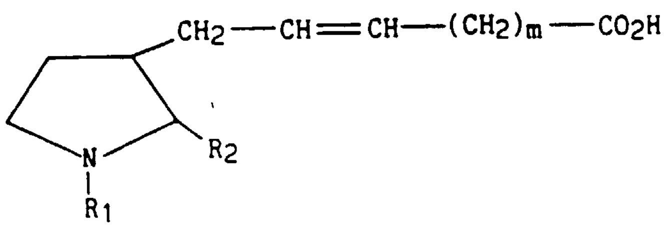

(I/a)

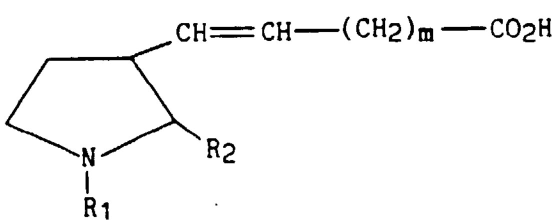

(I/b)

wherein $R_1$, $R_2$ and m are as defined for formula (I),

which compounds of formula (I/a) or (I/b)

- are esterified, if desired, to yield the corresponding compounds of formulae ($I/a_1$) and ($I/b_1$) :

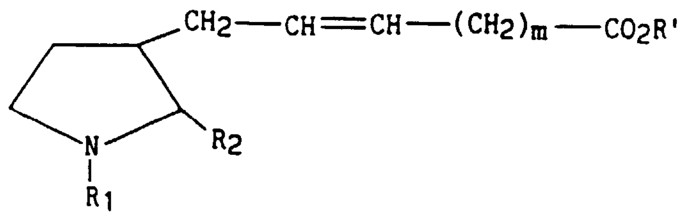

(I/$a_1$)

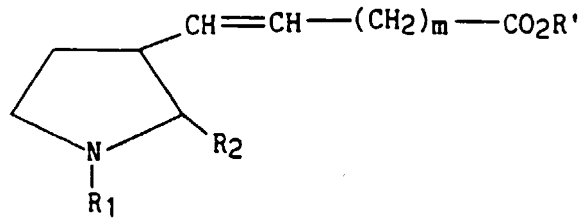

(I/$b_1$)

wherein $R_1$ and $R_2$ are as defined for formula (I) and R' represents a straight-chained or branched ($C_1$-$C_6$)-alkyl group,

- of which the double bond is optionally reduced by catalytic hydrogenation to yield the compound of formula (I/c), a particular case of compounds of formula (I),

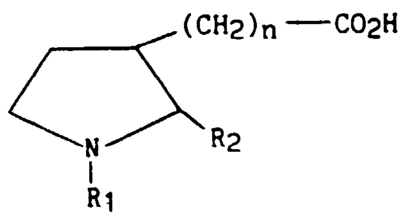

(I/c)

wherein $R_1$, $R_2$ and n are as defined for formula (I), and which is converted, if desired, into the corresponding ester of formula (I/$c_1$) :

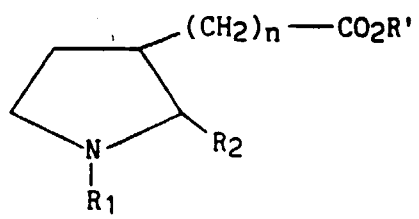

(I/$c_1$)

wherein $R_1$ and $R_2$ are as defined for formula (I) and R' represents a straight-chained or branched ($C_1$-$C_6$)-alkyl group,

which compounds of formulae (I/a), (I/$a_1$), (I/b), (I/$b_1$), (I/c), (I/$c_1$) constitute the entirety of the compounds of formula (I),

- which, when $R_2$ represents a phenyl nucleus substituted by a straight-chained or branched ($C_1$-$C_6$)-alkoxy group, may be converted, if desired, into compounds of formula (I) wherein $R_2$ represents a phenyl nucleus substituted by a hydroxy group,
- which are purified, where necessary, according to a conventional method of purification,
- which are separated, where appropriate, into their isomers according to a conventional method of separation,
- and which are converted, if desired, into their addition salts with a pharmaceutically acceptable acid or base.

**7.** Pharmaceutical compositions comprising as active ingredient at least one compound according to any one of claims 1 to 5, alone or in combination with one or more pharmaceutically acceptable, inert, non-toxic carriers.

**8.** Pharmaceutical composition according to claim 7 comprising at least one active ingredient according to any one of claims 1 to 5 for use as thromboxane $A_2$ receptor antagonists or as thromboxane $A_2$-synthase inhibitors.

**Claims for the following Contracting State : ES**

**1.** Process for the preparation of compounds of formula (I) :

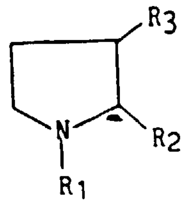

(I)

wherein :
$R_1$ represents :

- a straight-chained or branched ($C_1$-$C_6$)-alkyl group that is unsubstituted or substituted by a pyridin-2-yl, pyridin-3-yl or phenyl group (which is itself optionally substituted by one or more halogen atoms or groups straight-chained or branched ($C_1$-$C_6$)-alkyl, straight-chained or branched ($C_1$-$C_6$)-alkoxy, trihalomethyl),
- a phenyl group that is unsubstituted or substituted by one or more halogen atoms or groups straight-chained or branched ($C_1$-$C_6$)-alkyl, straight-chained or branched ($C_1$-$C_6$)-alkoxy, trihalomethyl,
- a pyridyl group,
- a phenylsulphonyl group that is unsubstituted or substituted at the phenyl nucleus by one or more halogen atoms or groups straight-chained or branched ($C_1$-$C_6$)-alkyl, straight-chained or branched ($C_1$-$C_6$)-alkoxy, trihalomethyl,
- a straight-chained or branched ($C_1$-$C_6$)-acyl group,
- a straight-chained or branched ($C_1$-$C_6$)-alkoxycarbonyl group,
- a benzoyl group (unsubstituted or substituted at the phenyl nucleus by one or more halogen atoms or groups hydroxy, alkyl, alkoxy, trifluoromethyl) or a pyridylcarbonyl group,
- an alkylaminocarbonyl or phenylaminocarbonyl group (unsubstituted or substituted at the phenyl nucleus by one or more halogen atoms or groups hydroxy, alkyl, alkoxy, trifluoromethyl),
- an acylamino or benzoylamino group,

$R_2$ represents :

- a phenyl group that is unsubstituted or substituted by one or more halogen atoms or groups straight-chained or branched ($C_1$-$C_6$)-alkyl, straight-chained or branched ($C_1$-$C_6$)-alkoxy, hydroxy, trihalomethyl,
- a pyridin-3-yl or pyridin-2-yl group,

$R_3$ represents any one of the following groupings :

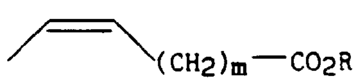

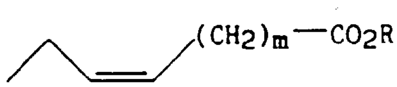

$-(CH_2)_n-CO_2R$

wherein

m is 2, 3 or 4,

n is 4, 5, 6 or 7,

and R represents a hydrogen atom or a straight-chained or branched (C1-C6)-alkyl group,

their enantiomers, diastereoisomers and epimers and also their addition salts with a pharmaceutically acceptable acid or base,
characterised in that there is used as starting material a pyrrolidine of formula (II) :

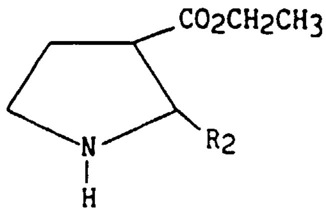

(II)

wherein $R_2$ is as defined for formula (I), in racemic form or which has previously been separated into its isomers according to a conventional method of separation,
which is reacted :

- either with lithium aluminium hydride in ether, to yield the pyrrolidine of formula (III) :

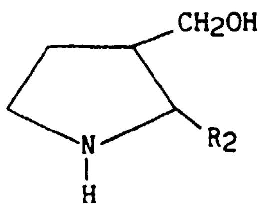

(III)

wherein $R_2$ is as defined for formula (I), which is then reacted with a halogenated compound of formula $R_1X$, wherein $R_1$ is as defined for formula (I) and X represents a halogen atom, in chloroform in the presence of triethylamine or in acetonitrile in the presence of potassium carbonate,
to yield the pyrrolidine of formula (IV) :

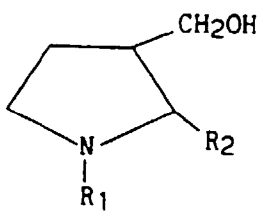

(IV)

wherein $R_1$ and $R_2$ are as defined for formula (I),

- or with a halogenated compound of formula $R_1X$ as defined above, to yield the pyrrolidine of formula (V) :

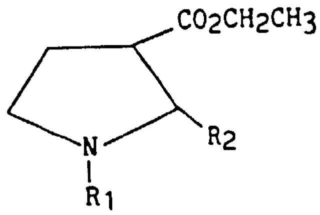

(V)

wherein $R_1$ and $R_2$ are as defined for formula (I),
which compound of formula (V), when it is in the form of a pair of enantiomers, may be converted, if desired, into its other pair of enantiomers by sodium ethanolate reaction in ethanolic medium,
which compound of formula (V) is reduced :

either a:

in the presence of two equivalents of diisobutylaluminium hydride in toluene,

to yield the pyrrolidine of formula (IV) defined above, which compound of formula (IV), whatever the method of synthesis by which it has been obtained, is converted by reaction with toluenesulphonyl chloride, in the presence of pyridine, into the corresponding tosylate (Ts) of formula (VI)

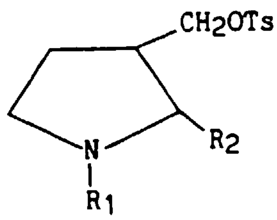

(VI)

wherein $R_1$ and $R_2$ are as defined for formula (I),
which is reacted with potassium cyanide in dimethyl sulphoxide medium,
to yield the pyrrolidine of formula (VII) :

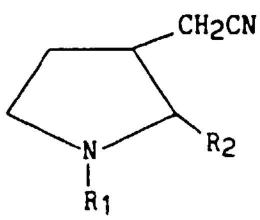

(VII)

wherein $R_1$ and $R_2$ are as defined for formula (I),
which is reduced with diisobutylaluminium hydride in toluene,
to yield the corresponding aldehyde of formula (VIII) :

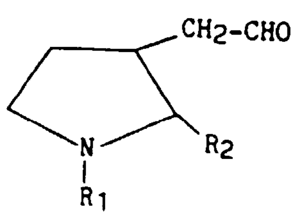

(VIII)

wherein $R_1$ and $R_2$ are as defined for formula (I),

or b:

in the presence of one equivalent of diisobutylaluminium hydride in toluene,

to yield the pyrrolidine of formula (IX) :

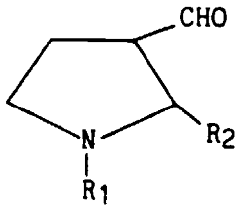

(IX)

wherein $R_1$ and $R_2$ are as defined for formula (I), which compounds of formula (VIII) or (IX) are reacted with a phosphorus ylid of formula (X), prepared by reaction of the corresponding phosphonium salt, in the presence of potassium tert-butanolate in tetrahydrofuran,

$$(C_6H_5)_3\,P = CH\text{-}(CH_2)_m\text{-}CO_2H \qquad (X)$$

wherein m is as defined for formula (I), to yield, respectively, the compounds of formula (I/a) or (I/b), a particular case of compounds of formula (I) :

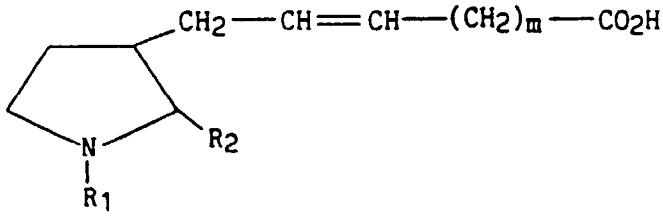

(I/a)

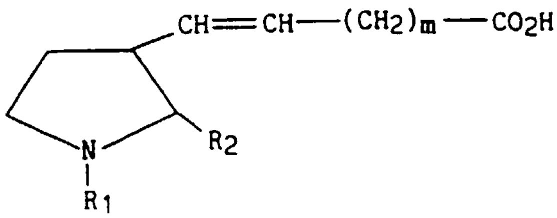

(I/b)

wherein $R_1$, $R_2$ and m are as defined for formula (I),

which compounds of formula (I/a) or (I/b)

- are esterified, if desired, to yield the corresponding compounds of formulae ($I/a_1$) and ($I/b_1$) :

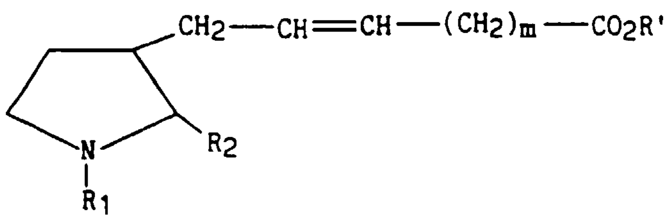

($I/a_1$)

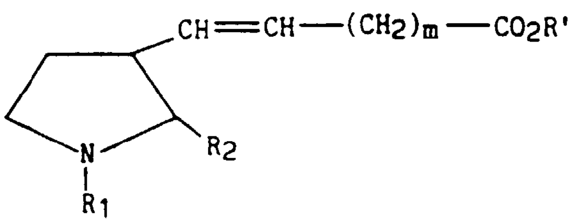

($I/b_1$)

wherein $R_1$ and $R_2$ are as defined for formula (I) and R' represents a straight-chained or branched ($C_1$-$C_6$)-alkyl group,

- of which the double bond is optionally reduced by catalytic hydrogenation to yield the compound of formula

(I/c), a particular case of compounds of formula (I),

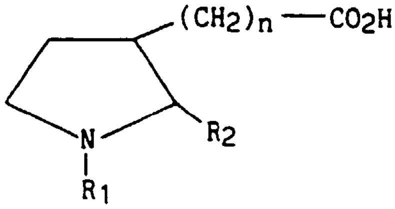

(I/c)

wherein $R_1$, $R_2$ and n are as defined for formula (I), and which is converted, if desired, into the corresponding ester of formula ($I/c_1$) :

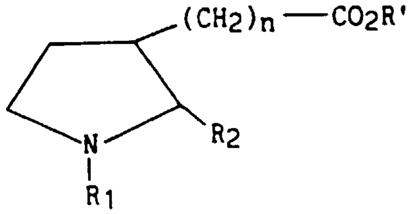

($I/c_1$)

wherein $R_1$ and $R_2$ are as defined for formula (I) and R' represents a straight-chained or branched ($C_1$-$C_6$)-alkyl group,

which compounds of formulae (I/a), ($I/a_1$), (I/b), ($I/b_1$), (I/c), ($I/c_1$) constitute the entirety of the compounds of formula (I),

- which, when $R_2$ represents a phenyl nucleus substituted by a straight-chained or branched ($C_1$-$C_6$)-alkoxy group, may be converted, if desired, into compounds of formula (I) wherein $R_2$ represents a phenyl nucleus substituted by a hydroxy group,
- which are purified, where necessary, according to a conventional method of purification,
- which are separated, where appropriate, into their isomers according to a conventional method of separation,
- and which are converted, if desired, into their addition salts with a pharmaceutically acceptable acid or base.

**2.** Process for the preparation of compounds of formula (I) according to claim 1, wherein wherein $R_1$ represents a substituted or unsubstituted phenylsulphonyl group, their enantiomers, diastereoisomers and epimers and also their addition salts with a pharmaceutically acceptable acid or base.

**3.** Process for the preparation of compounds of formula (I) according to claim 1, wherein $R_1$ represents a pyridin-3-ylmethyl group, their enantiomers, diastereoisomers and epimers and also their addition salts with a pharmaceutically acceptable acid or base.

**4.** Process for the preparation of compounds of formula (I) according to claim 1, wherein R2 represents a substituted or unsubstituted phenyl group, their enantiomers, diastereoisomers and epimers and also their addition salts with a pharmaceutically acceptable acid or base.

**5.** Process for the preparation of the compound of formula (I) according to any one of claims 1, 3 and 4 which is (4Z)-6-[(2α,3α)-1-[(pyridin-3-yl)methyl]-2-phenylpyrrolidin-3-yl]hex-4-enoic acid, its enantiomers, diastereoisomers and also its addition salts with a pharmaceutically acceptable acid or base.